# EUROPEAN PATENT SPECIFICATION

(11) **EP 3 139 951 B1**
(45) Date of publication and mention of the grant of the patent: **09.09.2020**
(21) Application number: 15727869.8
(22) Date of filing: 11.05.2015
(51) Int. Cl.: A61K 39/02, C07K 14/20, A61K 39/00

(54) **VACCINE FOR PREVENTION OF LYME BORRELIOSIS**
IMPFSTOFF ZUR PRÄVENTION VON LYME-BORRELIOSE
VACCIN POUR LA PRÉVENTION DE LA BORRÉLIOSE DE LYME

(30) Priority: 09.05.2014 CZ 20140320
(43) Date of publication of application: 15.03.2017
(73) Proprietor: Vyzkumny ústav veterinárního lékarství, v.v.i., 621 00 Brno (CZ); Univerzita Palackého v Olomouci, 771 47 Olomouc (CZ); Bioveta, A.S., 68323 Ivanovice na Hane (CZ); Ustav organicke chemie a biochemie AV CR, v.v.i., 166 10 Praha 6 (CZ); Fyzikální ústav AV CR, v.v.i., 18221 Praha 8 (CZ)
(72) Inventor: TURANEK, Jaroslav, 63800 Brno-Lesna (CZ); MASEK, Josef, 63800 Brno-Lesna (CZ); RASKA, Milan, 77900 Olomouc (CZ); WEIGL, Evzen, 77900 Olomouc (CZ); KRUPKA, Michal, 77900 Olomouc (CZ); BITTNER, Libor, 68201 Vyskov-Dedice (CZ); NEPERENY, Jiri, 68201 Vyskov-Nosalovice (CZ); VRZAL, Vladimir, 68323 Ivanovice na Hane (CZ); LEDVINA, Miroslav, 18600 Praha 8 (CZ); KRATOCHVILOVA, Irena, 18100 Praha 8-Troja (CZ)
(74) Representative: Hartvichova, Katerina
(86) International application number: PCT/CZ2015/000042
(87) International publication number: WO 2015/169271

(56) References cited:
- WO-A1-95/12676
- WO-A1-2011/143617
- WO-A1-2014/018274
- WO-A2-02/16422
- WO-A2-2007/065098
- WO-A2-2012/054580
- Michal Krupka ET AL: "Recombinant polyepitop-forming chimera protein derived from Borrelia burgdorferi OspC variants induces specific antibodies recognizing wide spectrum of antigenic variants of pathogenic Borreliae.", , 22 August 2013 (2013-08-22), XP055201147, Retrieved from the Internet: URL:http://www.frontiersin.org/10.3389/con f.fimmu.2013.02.00469/event_abstract [retrieved on 2015-07-08]
- EARNHART ET AL: "Construction and analysis of variants of a polyvalent Lyme disease vaccine: Approaches for improving the immune response to chimeric vaccinogens", VACCINE, ELSEVIER LTD, GB, vol. 25, no. 17, 5 April 2007 (2007-04-05) , pages 3419-3427, XP022022120, ISSN: 0264-410X, DOI: 10.1016/J.VACCINE.2006.12.051
- Christopher G. Earnhart ET AL: "An Octavalent Lyme Disease Vaccine Induces Antibodies That Recognize All Incorporated ospC Type-Specific Sequences", Human Vaccines, 1 November 2007 (2007-11-01), pages 281-289, XP055100831, Retrieved from the Internet: URL:http://www.landesbioscience.com/journa ls/vaccines/article/4661/ [retrieved on 2015-07-23]
- C. G. EARNHART ET AL: "Demonstration of OspC Type Diversity in Invasive Human Lyme Disease Isolates and Identification of Previously Uncharacterized Epitopes That Define the Specificity of the OspC Murine Antibody Response", INFECTION AND IMMUNITY, vol. 73, no. 12, 18 November 2005 (2005-11-18), pages 7869-7877, XP055199803, ISSN: 0019-9567, DOI: 10.1128/IAI.73.12.7869-7877.2005
- MICHAL K UPKA ET AL: "Enhancement of immune response towards non-lipidizedrecombinant OspC antigen by binding onto the surface of metallochelating nanoliposomes with entrapped lipophilic derivatives of norAbuMDP", JOURNAL OF CONTROLLED RELEASE, ELSEVIER, AMSTERDAM, NL, vol. 160, no. 2, 18 February 2012 (2012-02-18), pages 374-381, XP028512654, ISSN: 0168-3659, DOI: 10.1016/J.JCONREL.2012.02.017 [retrieved on 2012-02-22]
- NINA WRESSNIGG ET AL: "Safety and immunogenicity of a novel multivalent OspA vaccine against Lyme borreliosis in healthy adults: a double-blind, randomised, dose-escalation phase 1/2 trial", LANCET INFECTIOUS DISEASES, ELSEVIER LTD, US, vol. 13, no. 8, 1 August 2013 (2013-08-01) , pages 680-689, XP002714295, ISSN: 1473-3099, DOI: 10.1016/S1473-3099(13)70110-5 [retrieved on 2013-05-10]
- P NOEL BARRETT ET AL: "A novel multivalent OspA vaccine against Lyme borreliosis shows promise in Phase I/II studies", EXPERT REVIEW OF VACCINES, vol. 12, no. 9, 1 September 2013 (2013-09-01), pages 973-975, XP055218377, ISSN: 1476-0584, DOI: 10.1586/14760584.2013.824704
- DING W ET AL: "Structural identification of a key protective B-cell epitope in lyme disease antigen OspA", JOURNAL OF MOLECULAR BIOLOGY, ACADEMIC PRESS, UNITED KINGDOM, vol. 302, no. 5, 6 October 2000 (2000-10-06), pages 1153-1164, XP004472344, ISSN: 0022-2836, DOI: 10.1006/JMBI.2000.4119
- LUFT B J ET AL: "A NEW MULTI-TARGET OSPA-OSPC VACCINE FOR LYME DISEASE", ABSTRACTS OF THE INTERSCIENCE CONFERENCE ON ANTIMICROBIAL AGENTSAND CHEMOTHER, XX, XX, vol. 40, 17 September 2000 (2000-09-17), XP008004693,

## Description

### Field of Art

The present invention relates to the composition and combination of recombinant antigens and vaccine formulation to prevent Lyme borreliosis and tick-borne diseases. The vaccine is intended for human and veterinary use.

### Background Art

Lyme borreliosis is a chronic multi-system infectious disease and the most common arthropod-borne infectious disease found both in Europe and the United States. It is generally not fatal, but it has serious health consequences in a considerable percentage of cases. From this perspective, it is a serious social and economic issue. Its importance is shown by the frequency of publications in journals that focus on infectious diseases. From this point of view, Lyme borreliosis has assumed a position just behind acquired immunodeficiency syndrome, as described e.g. in Barbour AG, Fish D, Science. 1993 Jun 11;260(5114):1610-6*.*

Clinical symptoms of Lyme disease have been described by various authors already at the turn of the 19^{th} and 20^{th} centuries *(Bartůněk P. Historie. In: Bartůněk P. et al. Lymeská borelióza. Praha: Grada Publishing, 2006: 11),* but it was only in 1982 that the agent causing the reported signs was described - spirochete named *Borrelia burgdorferi (*Burgdorfer W, Barbour AG, Hayes SF, Benach JL, Grunwaldt E, Davis JP, Science. 1982; 216:1317-1319*).* Other closely related species were successively identified, and thus the complex of species known as *Borrelia burgdorferi* sensu lato (s. 1.) currently includes twelve species and it is possible that the final number has yet to be determined.

Three out of the described species, namely *Borrelia burgdorferi* sensu stricto (s. s.), *Borrelia garinii* and *Borrelia afzelii* mainly exert their effect as pathogens on the Eurasian continent. On the North American continent only *B. burgdorferi* sensu stricto causes the disease. The most common vectors of the species *B. burgdorferi* are ticks of the genus Ixodes, but blood-sucking insects might most likely also transmit the infection, even though convincing evidence for this phenomenon is still lacking. The major natural hosts are small rodents, but also birds and deer. They thus represent the natural reservoir of this infection. Humans are considered dead-end hosts in the chain of this infection in the sense that while becoming infected, they do not spread the infection further. Currently, Lyme borreliosis is the most common zoonotic disease in Europe and North America. Moreover, its incidence is on the increase.

The clinical course of Lyme disease can generally be divided into three phases. However, not all infected subjects will have all the symptoms. The first phase of the disease presents a picture of flu-like symptoms, often accompanied by cutaneous manifestations at the site of the bite of an infected tick. Lyme disease causes typical skin conditions termed *erythema migrans.* If untreated and not eliminated by the host's immune system, the infection develops towards the next phase presented by arthritis, neurological, dermal, rarely cardiac and ocular complications etc. In about 10% of patients regardless of antibiotic treatment, clinical symptoms can linger for a long period of time. A possible explanation for this is the induction of autoimmune processes.

Severe damage to the mammalian body, as well as difficulties with diagnosis and treatment of Lyme disease have provided a major stimulus for the development of a vaccine. Furthermore, the enthusiasm in a successful vaccine development is also supported by the findings that the humoral immune response is sufficient for protection against this microbial infection.

From a microbiological point of view, microorganisms from the *Borrelia burgdorferi* s.l. species complex are characterized by a spiral form, which is determined mainly by flagella residing in the periplasmic space. The surface of Borrelia consists of *OspA, OspB, OspC, OspD, OspE* and *OspF* lipoproteins, or others, of which some may serve as target for protective immune response (Kumaru OS, Schulze RJ, Rodnin MV, Ladokhin AS, Zuckert WR, J Bacteriol. 2011 Jun; 193(11):2814-25*;* Livey I, O'Rourke M, Traweger A, Savidis-Dacho H, Crowe BA, Barrett PN, Yang X, Dunn JJ, Luft BJ, Clin Infect Dis. 2011 Feb;52 Suppl 3:s266-70*;* Chen S, Zuckert WR., J Bacteriol. 2011 Dec;193(23):6724-32*;* Tilly K, Bestor A, Rosa PA, Mol Microbiol. 2013 Jul;89(2):216-27*).*

The potential for the induction of a protective immune response has been described e.g. for the "outer surface" proteins OspA, OspB, OspC, fibronectin-binding protein BBK32 and decorin-binding proteins DbpA and DbpB *(*Gilmore RD 1996 Infect Immun 64:2234-2239*).* Outer surface proteins are generally encoded by genes localized on plasmids and are highly variable within a species and their expression is regulated according to the conditions of the bacteria life cycle.

In the gut of ticks, predominantly OspA and OspB proteins are expressed on the surface of Borrelia where they function as adhesion molecules. Once the ticks feed on warm-blooded hosts, the expression of OspA and OspB proteins is downregulated and the expression of OspC is upregulated. Once Borrelia infection transits into the disseminated stage, expression of OspC is downregulated and VIsE protein appears on the surface of Borrelia. This protein has a high antigenic variation, which is preconditioned by gene recombination of the multi-domain VIsE gene *(*Obonyo M, Munderloh UG, Fingerle V, Wilske B, Kurtti TJ, J ClinMicrobiol. 1999 Jul;37(7):2137-41*;* Xu Q, McShan K, Liang FT, Microbiology. 2008 Nov;154(Pt 11):3420-9. doi: 0.1099/mic.0.2008/019737-0*).*

The OspA protein was the first described member of outer surface lipoproteins *(*Barbour et al, 1983 Infect Immun. 41 (2): 795-804*).* OspA has a molecular weight of 31 kDa. Together with OspB protein, this protein is expressed on the surface of Borrelia in the midgut of the tick *(*Howe et al., 1985 Science. 227 (4687): 645-6*).* The main function of OspA protein consists in binding to TROSPA glycoprotein present in epithelial cells of the tick gut. Its expression is significantly downregulated during tick feeding. Another proposed function of OspA is the binding of plasminogen from the mammalian host blood. In analogy with some other microorganisms, the phenomenon of plasminogen binding by OspA antigen is described as the virulence factor *(*Lahteenmaki et al., 2001 FEMS Microbiol Rev. 25(5):531-52).

The OspC protein is a promising candidate for a preventive vaccine against Lyme borreliosis. It was identified in 1992 as the second antigen (after OspA) which induces a protective immune response (Preac-Mursic, Wilske et al. 1992 Infection 20(6):342-9). The OspC gene is located on a 27-kb circular plasmid *(*Marconi, RT, Samuels, DS and Garon, CF (1993) J.Bacteriol., 175, 926±932*).* It is a lipoprotein consisting of 210 amino acids with a molecular weight of 22-23 kDa. It is constituted predominantly of alpha helical structures connected by loops. The triacylglycerol lipid motif allows anchoring to the cell membrane. The protective effect of OspC immunization of experimental animals was confirmed after its administration in the early 1990s. However, the protein variability has been shown to be a main obstacle for vaccine development *(*Preac-Mursic V, Wilske B, Patsouris E et al. 1992 Infection 20:342-349*).* Gradually, 38 structural variants of OspC antigens were described. Some of them appear more frequently in Borrelia microorganisms causing systemic diseases in humans. Clones carrying these antigens are therefore referred to as invasive *(*Seinost, G., Dykhuizen, D.E., Dattywyler, R.J, Golde, W.T., Dunn, J.J., Wang,I.-N., Wormser, G.P., Schriefer. M. E. and Lufl, B. J. 1999 Infect. Immun., 67, 3518±3524*).*

The first veterinary vaccine against borreliosis was developed for dogs in the United States in 1990. It was a whole-cell vaccine based on bacterins released from microorganisms after homogenisation of the culture *(*Levy SA, Lissman BA, Ficke CM, 1993 J Am Vet Med Assoc. Jun 1;202(11):1834-8*;* Wormser, GP, 1995 Ann Intern Med 123,627 - 629*).*

Experiments aiming to transfer protective immunity using sera from animals immunized with individual outer surface proteins, indicated feasibility of an effective subunite vaccine develoment. The outer surface proteins OspA, B, C and D have become the main candidate antigens for construction of such a vaccine. The OspA protein has been most intensively studied. The protective effect of this subunit vaccine was confirmed experimentally in mice, hamsters and rabbits *(*Fikrig E et al., 1990 Science Oct 26; 250(4980):553-6*;* Fikrig E et al., 1992 Infect Immun. Mar; 60(3):773-7*).* Immunization of experimental animals with OspA protein in the form of a purified recombinant lipidated protein administered parenterally prevents development of the infection after Borrelia challenge performed either by needle injection or by feeding the infected tick on immunized animals. It was also confirmed that infected ticks sucking animals which were immunized in this way lost their infectiosity *(*Keller D et al., 1994 JAMA. Jun 8;271(22): 1764-8*;* Fikrig E et al, 1992 ProcNatlAcadSci USA. Jun 15;89(12):5418-21*).* The international patent application WO 02/16422 discloses chimeric vaccines which, for example, are composed of fusion proteins comprising both OspC and OspA antigenic regions.

Therefore, the first commercial monovalent vaccine was based on the OspA antigen. The vaccine was approved by the US Food and Drug Administration (FDA) in 1998 *(*Steere AC et al, N Engl J Med. 1998 Jul 23;339(4):209-15*).* The peculiarity of this vaccine is the fact that the host-pathogen interaction takes place outside the host body - in the tick midgut. The drawback of this vaccine was its monovalent nature. The vaccine consists of the OspA protein from *Borrelia burgdorferi* sensu stricto. This genomic species is the dominant infectious agent of Lyme disease in North America and thus it may be effective for the US population. However, it is not optimal for European countries. The OspA antigen prepared from a strain of *Borrelia burgdorferi* sensu stricto cannot cover the variabile OspA antigens of the three genomic species on the European continent.

Ferritin II is a key protein which ensures homeostasis in blood-sucking ticks that receive huge amounts of iron in the form of hemoglobin from the host's blood. It has been demonstrated that vaccination of rabbits with recombinant tick Ferritin II protein significantly reduces tick feeding on vaccinated rabbits *(*Hajdusek et al. 2010 Vaccine 28, 2993-2998*).*

Change from live vaccines to safer but less immunogenic vaccines based on recombinant antigens requires new structurally defined adjuvants stimulating the antibody- and T-cell-mediated immunity. In the case of Borrelia infection, opsonizing and especially complement-activating antibodies are considered key mechanisms of host's protection. Vaccines approved for clinical use are mainly using aluminium hydroxide, aluminium phosphate or potassium alum as an adjuvant. These adjuvants induce predominantly a Th2-driven immune response associated with the formation of neutralizing antibodies. The only alternatives approved for human clinical usage, which partly potentiate the effector cell immunity are oil emulsions MF59 (Novartis) and AS03 (GSK). Both induce only the mild Thl immune response necessary for the formation of opsonizing and complement-activating antibodies. Such requirements are fully met by the original, fully synthetic, non-pyrogenic, molecular-based adjuvant derived from normuramyl glycopeptides. The combination of structural changes in the saccharide and peptide moieties eliminated the clinically intolerable high pyrogenicity generally associated with muramyl glycopeptides *(*Ledvina M., Jezek J., Saman D., Hribalova V.: Collect. Czech. Chem. Commun. 1998, 63, 590-598*;* Ledvina M., Zyka D., Jezek J., Trnka T., Saman D.: Collect. Czech. Chem. Commun. 1998, 63, 577-589*).* Due to the amphiphilic nature of these adjuvants, given by the presence of hydrophobic and polar/hydrophilic domains in their molecule, these substances are capable of forming stable micellar structures and can be efficiently incorporated into the lipid bilayer of liposome carrier systems *(*Turanek J., Masek J, Raska M, Ledvina M, in: Biomedical Science, Engineering and Technology, Application of Liposomes for Construction of Vaccines, InTech, January, 2012, 653-678*.).*

Liposomes, vesicles from phospholipid membranes represent biocompatible and multipurpose carriers for fomulation of targeted drugs and construction of recombinant vaccines. Liposomes are nanoparticulate systems approved by the FDA for use in human medicine. Their use as adjuvants was first described by Gregoriadis and Allison (Nature 252, 252, 1974*).* The use of metallochelating liposomes for the construction of recombinant vaccines has been described *(*Krupka et al. Journal of Controlled Release 160 (2012) 374-381*;* Masek et al. 151 (2011) 193-201*;* Masek et al. Analytical Biochemistry 408 (2011) 95-104*).*

### Disclosure of the Invention

The object of the invention is the vaccine for treatment and prevention of Lyme borreliosis for human and/or veterinary applications, which contains
- chimeric polyepitope recombinant antigen having a sequence having at least 95% identity with the sequence (SEQ ID NO. 7) 
1 MGSSHHHHHH SSGLVPRGSH MASMTGGQQM GRGSMKKCSE EFSTKLKDNH
51 AQLGIQGVTD ENAKKAILKA NAAGKDKGVE ELEKLSGSLE SLSKAAKEML
101 ANSVKKKCSE DFTKKLEGEH AQLGIEQVTD ENAKKAILIT DAAKDKGAAE
151 LEKLFKAVEN LAKAAKEMLA NSVKKKCSET FTNKLKEKHT DLGKEGVTDA
201 DAKEAILKTQ GTKTKGAEEL GKLFESVEVL SKAAKEMLAN SVKKKCSEEF
251 TAKLKGEHTD LGKEGVTDDN AKKAILKTNN DKTKGADELE KLFESVKQLS
301 KAAKEMLTNS VKKDCSEKFT TKLKDSHAEL GIQSVQDDNA KKAILKTHGT
351 KDKGAKELEE LFKSLESLSK AAQAALTNSV KKKCSEEFTN KLKSGHADLG
401 KQDATDDHAK AAILKTHATT DKGAKEFKDL FESVEGLLKA AQVALTNSVK
451 ELTSPVVAES PKKN;
- chimeric polyepitope recombinant antigen having a sequence having at least 95% identity with the sequence (SEQ ID NO. 15) 
1 MGSSHHHHHH SSGLVPRGSH MASMTGGQQM GRGSLKVTEG TVVLSKHIPN
51 SGEITVELND SNSTQATKKT GKWDSNTSTL TISVNSKKTK NIVFTKEDTI
101 TVQKYDSAGT NLEGNLEVKE GTVTLSKEIA KSGEVTVALN DTNTTQATKK
151 TGAWDSKTST LTISVNSKKT TQLVFTKQDT ITVQKYDSAG TNLEGTLKVT
201 EGTVTLSKNI SKSGEITVAL DDTDSSGNKK SGTWDSGTST LTISKNRTKT
251 KQLVFTKEDT ITVQNYDSAG TNLEGKTLKV TEGTVVLSKN ILKSGEITVA
301 LDDSDTTQAT KKTGKWDSKT STLTISVNSQ KTKNLVFTKE DTITVQKYDS
351 AGTNLEGKLV VKEGTVTLSK NISKSGEVSV ELNDTDSSAA TKKTAAWNSG
401 TSTLTITVNS KKTKDLVFTK ENTITVQQYD SNGTKLEGSL TVTEGTVTLS
451 KNISKSGEIT VALNDTETTP ADKKTGEWKS DTSTLTISKN SQKPKQLVFT
501 KENTITVQNY NRAGNALEGS LTVEAGTVTL SKNISESGEI TVELKDTETT
551 PADKKSGTWD SKTSTLTISK NSQKTKQLVF TKENTITVQK YNTAGTKLEG
601 SAVEITKLDE IKNALKKDLE ALKAALK
- and at least one adjuvant and/or Ferritin II.

At the N-terminal end, a sequence of six histidines (His tag) is preferably connected to the fusion protein, suitable for metallochelating binding to a liposomal carrier. Such a method of the recombinant protein fixation to the liposomal carrier ensures immunologically preferred orientation of the fusion protein so that the immunodominant C-terminal amino acid sequences are more accessible to the induction of immune response.

As disclosed herein, the polyepitope antigens and/or adjuvants and/or Ferritin II are linked to a micro- or nano-particulate carrier (i.e. a nanoparticulate carrier with particle size of up to 1 micrometre, and a microparticulate carrier with particle size of up to 100 micrometres) comprising a metallochelating component for binding of the above-mentioned components.

The carrier is preferably a liposome having an incorporated metallochelating lipid (e.g. DOGS- Ni-NTA (Avanti Polar Lipids, USA)) as a metallochelating binding component for binding the antigens or a molecular adjuvant and/or Ferritin II of the recombinant protein to the liposome surface. The carrier may also be a pharmaceutically acceptable polymer, such as copolymers of lactic and glycolic acids, polymers and copolymers based on acrylic and methacrylic acids, acrylates, methacrylates, e.g. hydroxymethylmethacrylate. The carrier may also be particles on the basis of inorganic materials, e.g. aluminum hydroxide, aluminum phosphate, potassium alum and silicate hydrogels.

The vaccine may further optionally contain additives, such as solvents, stabilizers, emulsifiers, carriers, etc.

The adjuvants may preferably be selected from the group comprising aluminium hydroxide, aluminium phosphate, potassium alum, molecular adjuvants on the basis of normuramyl glycopeptides described in WO 2009/115782 (general formulas I and II) or their glycosidic (especially methylglycosidic) forms (e.g. methylglycoside of *N*-acetylglucosamine for the stabilization of the hemiacetal form of the glycan moiety of the adjuvant by converting the glycan moiety to the acetal form), adjuvants based on the dispersion of particles consisting of polyacrylate polymer carriers (e.g. PetGelA).

Molecular adjuvants based on normuramyl glycopeptides are especially selected from the group comprising: 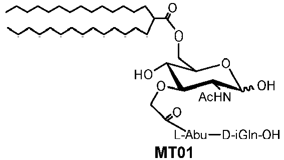 6-*O*-(2-Tetradecylhexadecanoyl)-*N*-acetylnormuramoyl-L-2-aminobutanoyl-D-isoglutamin e, 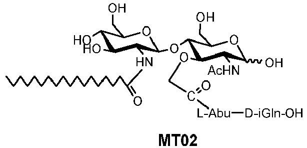 2-Deoxy-2-stearoylamino-β-D-glucopyranosyl-(1 →4)-*N*-acetylnormuramoyl-L-2-aminobutanoyl-D-isoglutamine, 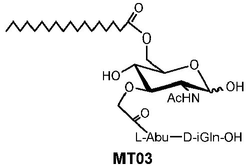 6-*O*-Stearoyl-*N*-acetylnormuramoyl-L-2-aminobutanoyl-D-isoglutamine, 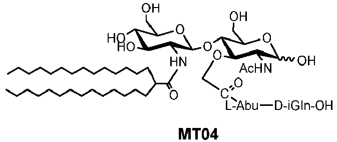 2-Deoxy-2-(2-tetradecylhexadecanoylamino)-β-D-glucopyranosyl-(1 →4)-*N*-acetylnormuramoyl-L-2-aminobutanoyl-D-isoglutamine, 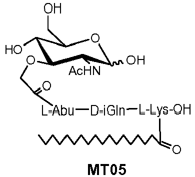 *N*-Acetylnormuramoyl-L-2-aminobutanoyl-D- isoglutaminyl-L-lysine(stearoyl), 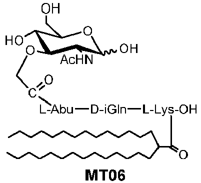 *N*-Acetylnormuramoyl-L-2-aminobutanoyl-D-isoglutaminyl-L-lysine(2-tetradecylhexadecanoyl), 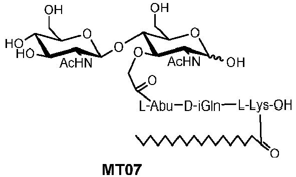 2-Acetamido-2-deoxy-β-D-glucopyranosyl-(1→4)-*N*-acetylnormuramoyl-L-2-aminobutanoyl-D- isoglutaminyl-L-lysine(stearoyl), 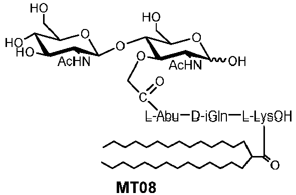 2-Acetamido-2-deoxy-β-D-glucopyranosyl-(1→4)-*N*-acetylnormuramoyl-L-2-aminobutanoyl-D- isoglutaminyl-L-lysine(2-tetradecylhexadecanoyl); and their glycosidic forms:
The sequences having at least 95% identity with the above-mentioned sequences can be derived in particular by shortening or extension of the sequence length by removing or additing amino acids on one or both ends of the sequence, and/or eventually by point mutations, without affecting the immunogenicity of the antigens.

The OspC antigens from which the immunodominant antigens and the terminal region are derived are:
NP_047005 - gill 1497007: **OspC group A** (B31MI) from *B. burgdorferi* (SEQ ID NO. 1) 
1 MKKNTLSAIL MTLFLFISCN NSGKDGNTSA NSADESVKGP NLTEISKKIT
51 DSNAVLLAVK EVEALLSSID EIAAKAIGKK IHQNNGLDTE NNHNGSLLAG
101 AYAISTLIKQ KLDGLKNEGL KEKIDAAKKC SETFTNKLKE KHTDLGKEGV
151 TDADAKEAIL KTNGTKTKGA EELGKLFESV EVLSKAAKEM LANSVKELTS
201 PVVAESPKKP
EF053525 - gill 17574163: **OspC group B** (LDP73) from *B. burgdorferi* (SEQ ID NO. 2) 
1 NNSGKDGNTS ANSADESVKG PNLTEISKKI TDSNAVLLAV KEVEALLSSI
51 DELAKAIGKK IKNDGSLDNE ANRNESLLAG AYTISTLITQ KLSKLNGSEG
101 LKEKIAAAKK CSEEFSTKLK DNHAQLGIQG VTDENAKKAI LKANAAGKDK
151 GVEELEKLSG SLESLSKAAK EMLANSVKEL TSPVVAESPK KP
AF467874 - gi|23507072: **OspC group I** (B331) from *B. burgdorferi* (SEQ ID NO. 3) 
1 MKKNTLSAIL MTLFLFISCN NSGKDGNTSA NSADESVKGP NLTEISKKIT
51 ESNAVVLAVK EVETLLTSID ELAKAIGKKI KNDVSLDNEA DHNGSLISGA
101 YLISTLITKK ISAIKDSGEL KAEIEKAKKC SEEFTAKLKG EHTDLGKEGV
151 TDDNAKKAIL KTNNDKTKGA DELEKLFESV KNLSKAAKEM LTNSVKELTS
201 PVVAESPKKP
EF053517 - gill 17574147: **OspC group K** (LDP74) from *B. burgdorferi* (SEQ ID NO. 4) 
1 NNSGKDGNTS ANSADESVKG PNLTEISKKI TESNAVVLAV KEIETLLASI
51 DELATKAIGK KIQQNGGLAV EAGHNGTLLA GAYTISKLIT QKLDGLKNSE
101 KLKEKIENAK KCSEDFTKKL EGEHAQLGIE NVTDENAKKA ILITDAAKDK
151 GAAELEKLFK AVENLAKAAK EMLANSVKEL TSPIVAESPK KP
YPJ363261 - gi|51038597: **OspC group PBi** from *B. garinii* (SEQ ID NO. 5) 
1 MKKNTLSAIL MTLFLFISCN NSGGDSASTN PDESAKGPNL TVISKKITDS
51 NAFLLAVKEV EALLSSIDEL SKAIGKKIKN DGTLDNEANR NESLIAGAYE
101 ISKLITQKLS VLNSEELKEK IKEAKDCSEK FTTKLKDSHA ELGIQSVQDD
151 NAKKAILKTH GTKDKGAKEL EELFKSLESL SKAAQAALTN SVKELTNPVV
201 AETPKKP
YP_709265 - gill 11074118: **OspC group PKo** from *B. afzelii* (SEQ ID NO. 6)

1 MKKNTLSAIL MTLFLFISCN NSGKGGDSAS TNPADESAKG PNLTEISKKI
51 TDSNAFVLAV KEVETLVLSI DELAKKAIGQ KIDNNNGLAA LNNQNGSLLA
101 GAYAISTLIT EKLSKLKNLE ELKTEIAKAK KCSEEFTNKL KSGHADLGKQ
151 DATDDHAKAA ILKTHATTDK GAKEFKDLFE SVEGLLKAAQ VALTNSVKEL
201 TSPVVAESPK KP

From these OspC antigens, regions corresponding to α3-L5-α4-L6-α5 segments of particular OspC proteins were selected, see the following table, and the C' terminal segment derived from OspC PKo.

| Designation in GeneBank database | The selected sequence segment - AA No. | Classification of OspC antigen according to group |
|---|---|---|
| NP_047005 -gi\| 11497007 | **128-196** | *B. burgdorferi* group **A** (B31MI) |
| EF053525 -gill 17574163 | **109-178** | *B. burgdorferi* group **B** (LDP73) |
| AF467874 - gi\|23507072 | **128-196** | *B. burgdorferi* group **I** (B331) |
| EF053517 - gill 17574147 | **110-178** | *B. burgdorferi* group **K** (LDP74) |
| YP_063261 - gi\|51038597 | **125-193** | *B. garinii* group **PBi** |
| YPJ709265 - gi\|1 11074118 | **130-211** | *B. afzelii* group **Pko** |

In a preferred embodiment, the recombinant polyepitope chimeric antigen based on OspC antigens contains three peptide sections attached at the N' terminus: 1) hexa His-tag allowing protein purification by Ni-NTA affinity chromatography and production of metalchellating proteoliposomes, 2) thrombin cleavage site enabling the removal of the His tag from the recombinant protein for the immunodetection (ELISA) applications detecting the levels of antigen-induced specific antibodies in immunized individuals, 3) T7 epitope allowing easy detection of the recombinant protein during the biotechnological production and purification using the anti-T7 antibody.

The OspA antigens from which the immunodominant antigens and the terminal region are derived are:
>X80257.1 - gi|258153: **OspA-PBi** from *B. garinii* (SEQ ID NO. 8) 
1 MKKYLLGIGL ILALIACKQN VSSLDEKNSV SVDLPGEMKV LVSKEKDKDG
51 KYSLMATVDK LELKGTSDKS NGSGTLEGEK SDKSKAKLTI SEDLSKTTFE
101 IFKEDGKTLV SKKVNSKDKS SIEEKFNAKG ELSEKTILRA NGTRLEYTEI
151 KSDGTGKAKE VLKDFALEGT LAADKTTLKV TEGTVVLSKH IPNSGEITVE
201 LNDSNSTQAT KKTGKWDSNT STLTISVNSK KTKNIVFTKE DTITVQKYDS
251 AGTNLEGNAV EIKTLDELKN ALK
>S48322 - gi|258151: **OspA-PKo** from *B. afzelii* (SEQ ID NO. 9) 
1 MKKYLLGIGL ILALIACKQN VSSLDEKNSA SVDLPGEMKV LVSKEKDKDG
51 KYSLKATVDK IELKGTSDKD NGSGVLEGTK DDKSKAKLTI ADDLSKTTFE
101 LFKEDGKTLV SRKVSSKDKT STDEMFNEKG ELSAKTMTRE NGTKLEYTEM
151 KSDGTGKAKE VLKNFTLEGK VANDKVTLEV KEGTVTLSKE IAKSGEVTVA
201 LNDTNTTQAT KKTGAWDSKT STLTISVNSK KTTQLVFTKQ DTITVQKYDS
251 AGTNLEGTAV EIKTLDELKN ALK
>X80251 - gi|984067: **OspA-PHei** from *B. burgdorferi* (SEQ ID NO. 10) 
1 MKKYLLGIGL ILALIACKQN VSSLDEKNSV SVDLPGGMKV LVSKEKDKDG
51 KYSLMATVEK LELKGTSDKN NGSGTLEGEK TDKSKVKLTI AEDLSKTTFE
101 IFKEDGKTLV SKKVTLKDKS STEEKFNEKG EISEKTIVRA NGTRLEYTDI
151 KSDKTGKAKE VLKDFTLEGT LÄADGKTTLK VTEGTVTLSK NISKSGEITV
201 ALDDTDSSGN KKSGTWDSGT STLTISKNRT KTKQLVFTKE DTITVQNYDS
251 AGTNLEGKAV EITTLKELKN ALK
>X80252 - gi|984085:**OspA-TN**, **N34, VS307, G25** from *B. garinii,* **PWudll** from *B. burgdorferi* (SEQ ID NO. 11) 
1 MKKYLLGIGL ILALIACKQN VSSLDEKNSV SVDLPGGMTV LVSKEKDKDG
51 KYSLEATVDK LELKGTSDKN NGSGTLEGEK TDKSKVKLTI ADDLSQTKFE
101 IFKEDGKTLV SKKVTLKDKS STEEKFNEKG ETSEKTIVRA NGTRLEYTDI
151 KSDGSGKAKE VLKDFTLEGT LAADGKTTLK VTEGTVVLSK NILKSGEITV
 
201 ALDDSDTTQA TKKTGKWDSK TSTLTISVNS QKTKNLVFTK EDTITVQKYD
251 SAGTNLEGKA VEITTLKELK DARK
>X80182 - gi|984069: **OspA-PKa** from *B. burgdorferi* (SEQ ID NO. 12) 
1 MKKYLLGIGL ILALIACKQN VSSLDEKNSV SVDLPGEMKV LVSKEKNKDG
51 KYDLIATVDK LELKGTSDKN NGSGVLEGVK ADKSKVKLTI SDDLGQTTLE
101 VFKEDGKTLV SKKVTSKDKS STEEKFNEKG EVSEKIITRA DGTRLEYTGI
151 KSDGSGKAKE VLKGYVLEGT LTAEKTTLVV KEGTVTLSKN ISKSGEVSVE
201 LNDTDSSAAT KKTAAWNSGT STLTITVNSK KTKDLVFTKE NTITVQQYDS
251 NGTKLEGSAV EITKLDEIKN ALK
>X80256 - gi|984079: **OspA-PBr** from *B. garinii* (SEQ ID NO. 13) 
1 MKKYLLGIGL ILALIACKQN VSSLDEKNSV SVDLPGGMKV LVSKEKDKDG
51 KYSLMATVEK LELKGTSDKS NGSGVLEGEK ADKSKAKLTI SQDLNQTTFE
101 IFKEDGKTLV SRKVNSKDKS STEEKFNDKG KLSEKVVTRA NGTRLEYTEI
151 KNDGSGKAKE VLKGFALEGT LTDGGETKLT VTEGTVTLSK NISKSGEITV
201 ALNDTETTPA DKKTGEWKSD TSTLTISKNS QKPKQLVFTK ENTITVQNYN
251 RAGNALEGSP AEIKDLAELK AALK
>X80254 - gi|984081: **OspA-T25** from *B. garinii* (SEQ ID NO. 14)

1 MKKYLLGIGL ILALIACKQN VSSLDEKNSV SVDLPGEMKV LVSKEKDKDG
51 KYSLEATVDK LELKGTSDKN NGSGVLEGVK AAKSKAKLTI ADDLSQTKFE
101 IFKEDGKTLV SKKVTLKDKS STEEKFNDKG KLSEKVVTRA NGTRLEYTEI
151 QNDGSGKAKE VLKSLTLEGT LTADGETKLT VEAGTVTLSK NISESGEITV
201 ELKDTETTPA DKKSGTWDSK TSTLTISKNS QKTKQLVFTK ENTITVQKYN
251 TAGTKLEGSP AEIKDLEALK AALK

From the above-listed OspA antigens, the regions corresponding to the C-terminal barrel-shaped region consisting of β14 to β21 sheets of each of the above mentioned OspA, see the following table, and the C-terminal *α*-helix of the OspA protein were selected. The chimeric OspA polyepitope does not contain sequences that were identified in previous studies as potentially cross-(auto)reactive with human LFA-1 protein (amino acid region 165-173).

| Designation in GeneBank database | Sequence region in amino acids | Classification of OspC antigen according to group |
|---|---|---|
| X80257.1 - gi\|258153 | **178-258** | *B. garinii* **PBi** |
| S48322 - gi\|258151 | **178-258** | *B. afzelii* **PKo** |
| X80251 - gi\|984067 | **179-258** | *B. burgdorferi* **PHei** |
| X80252 - gi\|984085 | **178-259** | *B. garinii* **TN, N34, VS307, G25** *B. burgdorferi* **PWudll** |
| X80182 - gi\|984069 | **179-258** | *B. burgdorferi* **PKa** |
| X80256 - gi 984079 | **179-259** | *B. garinii* **PBr** |
| X80254 - gi\|984081 | **179-259** | *B. garinii* **T25** |

In a preferred embodiment, the recombinant chimeric fusion protein based on OspA antigens has three peptide sections attached at the N' terminus: 1) hexa His-tag allowing protein purification by Ni-NTA affinity chromatography and preparation of metallochelating proteoliposomes, 2) thrombin cleavage site enabling removal of His-tag from the recombinant protein for the immunodetection ELISA applications detecting the levels of antigen-induced specific antibodies in immunized individuals, 3) T7 epitope allowing easy detection of the recombinant protein during biotechnological production and purification using the anti-T7 antibody.

The vaccine is able to elicit immunity specific to required Borrelia strains, is easier to produce in comparison with any vaccine known in the art, allows easy formulation of vaccine with various ratios of OspA and OspC polyepitope antigens. Individual epitopes, due to the oriented assembly, are not sterically hindered, which is further enhanced by their location on separate fusion proteins.

Adjuvants such as aluminium salts or adjuvants based on polymer polyacrylate carriers (e.g. PetGelA) are especially suitable for veterinary medicine. Molecular adjuvants based on normuramyl glycopeptides are particularly suitable for use in human medicine, especially in combination with liposomal carriers. Also aluminium adjuvants are advantageous for human medicine.

### Brief description of Drawings

**Fig. 1** A three-dimensional predictive model of the structure of the chimeric OspA polyepitope (polyOspA) according to Example 1. The prediction was performed using Phyre 2 programme.

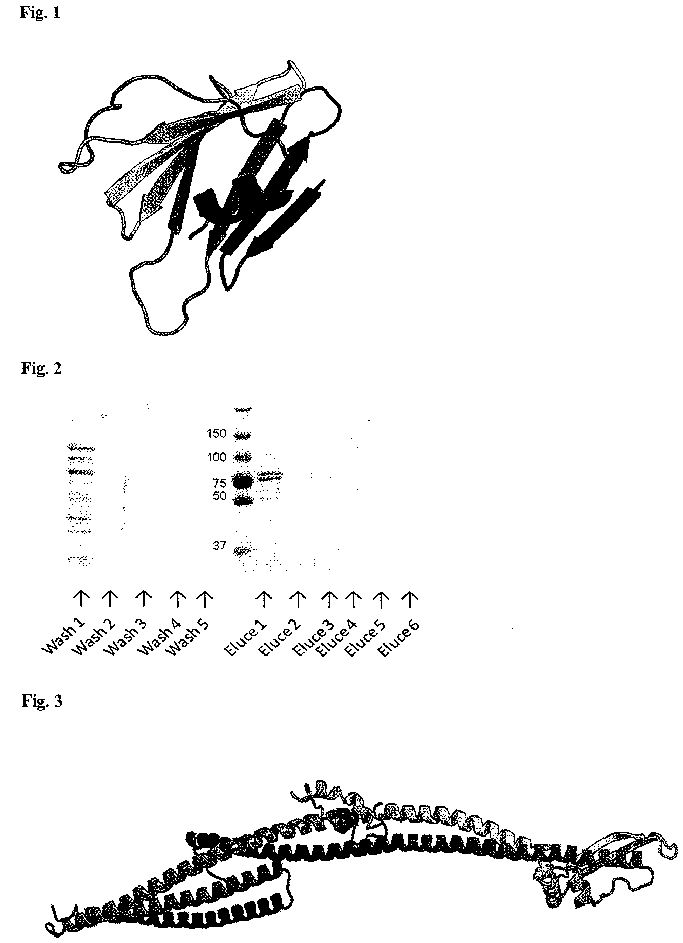

**Fig. 2** Results of recombinant chimeric OspA (polyOspA) polyepitope purification according to Example 1, by using affinity chromatography with Ni-NTA agarose. Bacteria expressing polyOspA were lysed under native conditions and the lysate was loaded onto a Ni-NTA column, and the column was then washed sequentially with a buffer which contained increasing concentrations of imidazole. The wash fractions (wash 1 - 10 mM imidazole, wash 2 - 30 mM imidazole, wash 3 - 40 mM imidazole, wash 4 - 60 mM imidazole, wash 5 - 80 mM imidazole) and the elution fractions performed with the buffer with imidazole concentration of 250 mM formed by three column volumes (Elution 1 to Elution 6) were loaded on 10% SDS-PAGE, and separated in an electric field. The gel was then stained with Coomassie Brilliant Blue.
**Fig. 3** A three-dimensional predictive model of the structure of the chimeric OspC polyepitope (polyOspC) according to Example 2. Prediction was performed using Phyre 2 program.

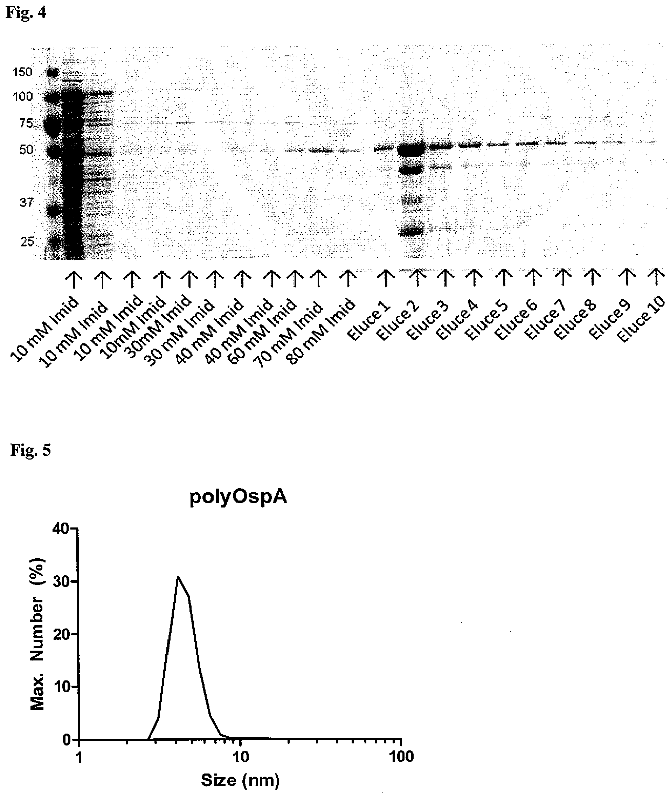

**Fig. 4** Results of recombinant chimeric OspC (polyOspC) polyepitope purification according to Example 2, by using affinity chromatography with Ni-NTA agarose. Bacteria expressing polyOspC were lysed under native conditions and the lysate was loaded onto a Ni-NTA column, and the column was then washed sequentially with buffer with increasing concentrations of imidazole. The wash fractions identified by the concentration of imidazole 10 mM to 80 mM imid and elution fraction, carried out with buffer containing imidazole at concentration of 250 mM consisting of 1.5 column volume (Elution 1 to Elution 10) were loaded on 10% SDS-PAGE and separated in an electric field. The gel was then stained with Coomassie Brilliant Blue.
**Fig. 5** Distribution of sizes of OspA polyepitope (polyOspA) (Example 3). The size of polyOspA (200 µg/ml in PBS) was measured using a dynamic light scattering on an apparatus ZetaSizer ZS (Malvern, UK).
**Fig. 6** Diagram of thermal stability of polyOspA antigen (Example 3). The thermal stability of polyOspA (200 µg/ml in PBS) was measured using a dynamic light scattering on an apparatus ZetaSizer ZS (Malvern, UK).
**Fig. 7** Size distribution of OspC polyepitope (polyOspC) (Example 3). The size of polyOspC (200 µg/ml in PBS) was measured using a dynamic light scattering on an apparatus ZetaSizer ZS (Malvern, UK).
**Fig. 8** Diagram of temperature stability of polyOspC antigen (Example 3). The temperature stability of polyOspC (200 µg/ml v PBS) was measured using a dynamic light scattering on an apparatus ZetaSizer ZS (Malvern, UK).
**Fig. 9** The effect of His-tag position at the C- and N-terminus on thermal denaturation of polyOspC antigen (Example 3). The characteristics were obtained by differential scanning calorimetry (protein concentration 1 mg/ml in PBS). A) polyOspC His tag at the N-terminus; B) polyOspC His-tag at the C-terminus.
**Fig. 10** Characteristics of thermal transition of polyOspA antigen (Example 3). Characteristics were obtained by differential scanning calorimetry (protein concentration 1 mg/ml PBS). PolyOspA His-tag at the N-terminus.

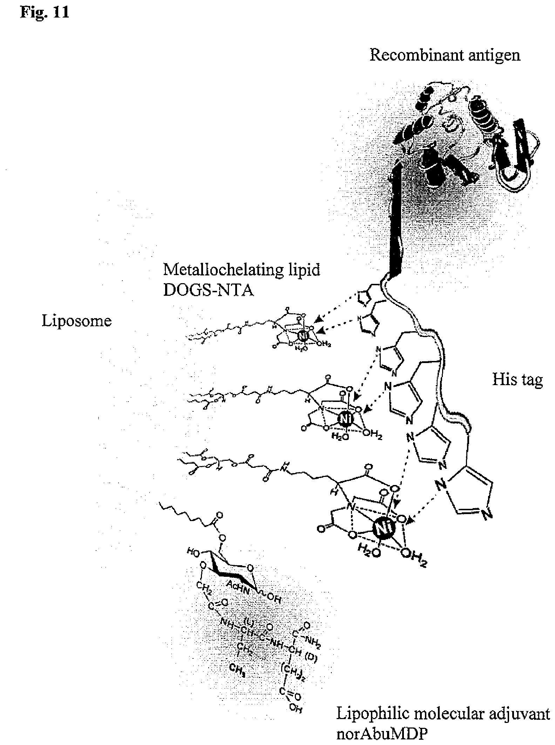

**Fig. 11** Diagram of structure of the liposomal component of a vaccine on the basis of metallochelating nanoliposomes with a molecular adjuvant (Example 4). On the left, there is a cut-out from the liposomal surface with DOGS-NTA protruding into the middle of the image (for antigen binding to the liposome surface) and, at the bottom of the picture, a non-pyrogenic lipophilic derivative of muramyl dipeptide (norAbuMDP) as an adjuvant. At the top right hand corner, there is a recombinant protein with a tag of 6 histidines, which non-covalently bind to the liposome surface via complexes of nickel (Ni) on a metallochelating DOGS-NTA lipid.
**Fig. 12** Demonstration of the binding of a recombinant antigen to metallochelating liposomes by changing the distribution of liposome sizes after binding of the polyepitope antigen (polyOspC) (Example 4). Change in the size distribution was measured using a ZetaSizer ZS (Malvern, UK). The inserted picture from TEM shows a liposome without a bound protein (A) and polyOspC molecules bound to the surface of the liposome (white arrows) and protein detection by immunolabelling with gold nanoparticles (black arrows) (B).

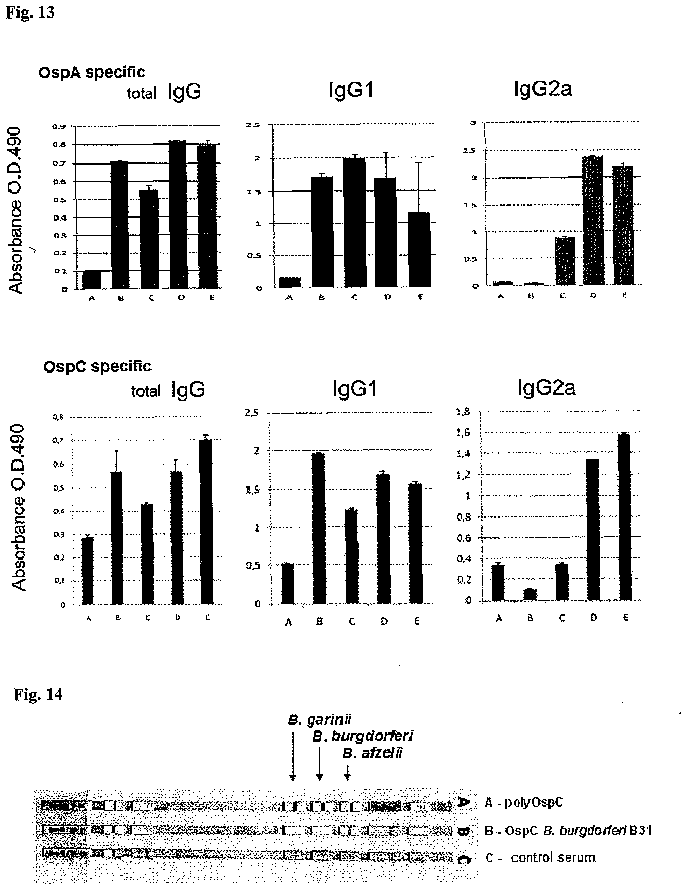

**Fig. 13** Results of the determination of serum antibodies in mice immunized with the polyepitopes polyOspA and polyOspC (Example 5). Reactivity of the antibodies was determined by ELISA. On the bottom of the ELISA panel, recombinant polyOspA (upper row of panels) or polyOspC was bound (lower row panels). Mouse sera were diluted identically and then dispensed into each well. Binding of serum antibody specifically reactive with OspA or OspC has been demonstrated by secondary antibody against total anti-mouse IgG or IgGl or IgG2a labelled peroxidase and then by colour reaction with orthophenylene diamine. Recombinant polyepitopes polyOspA and polyOspC used for immunization have an N-terminal His-tag.
   A. polyOspC or polyOspA
   B. polyOspC or polyOspA (adjuvant: Alum)
   C. polyOspC or polyOspA (adjuvant: Montanid - PET GEL A)
   D. polyOspC or polyOspA (adjuvant: metallochelating liposomes with a molecular adjuvant MT06)
   E. polyOspC or polyOspA (adjuvant: metallochelating liposomes with a molecular adjuvant MT06)
**Fig. 14** Reaction of sera from mice immunized by polyOspC with wild OspC proteins isolated from different Borrelia species (Example 5). Sera from mice immunized with recombinant OspC proteins with N-terminal attached His-tag was tested with diagnostic strips (Anti-Borrelia EUROLINE-RN-AT (IgM) containing areas with OspC of all three pathogenic strains - *B. garinii, B. burgdorferi, B. afzelii.* A - serum from a mouse immunized with a chimeric OspC polyepitope (polyOspC), B - serum from a mouse immunized with a recombinant OspC from *B. burgdorferi* B31, C - control - serum from a mouse vaccinated with recombinant antigen-labelled HIS from *Candida albicans* hsp90.
   A - chimeric OspC polyepitope (polyOspC)
   B - OspC from *B. burgdorferi* B31
   C - control serum (negative)

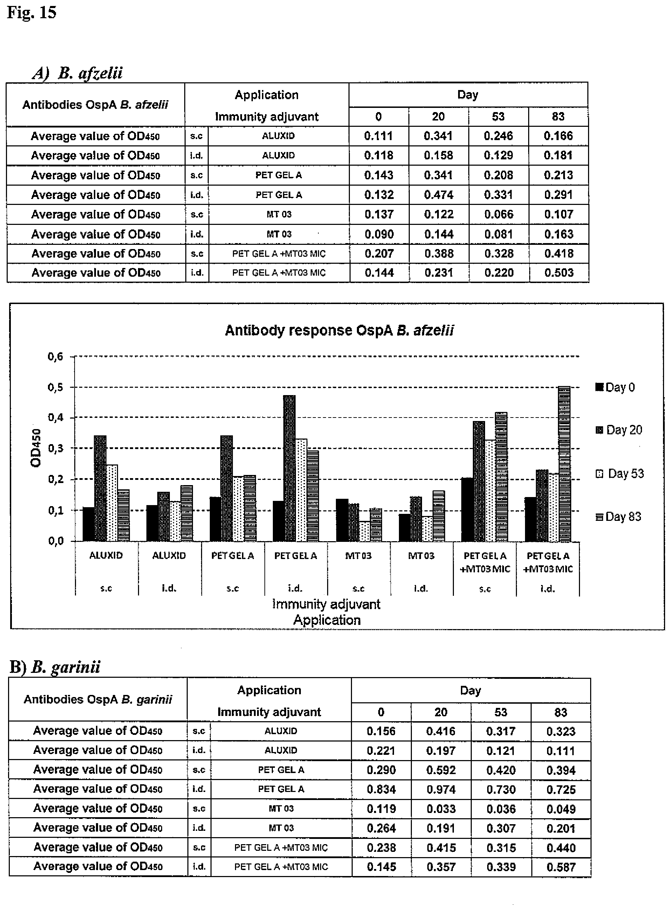

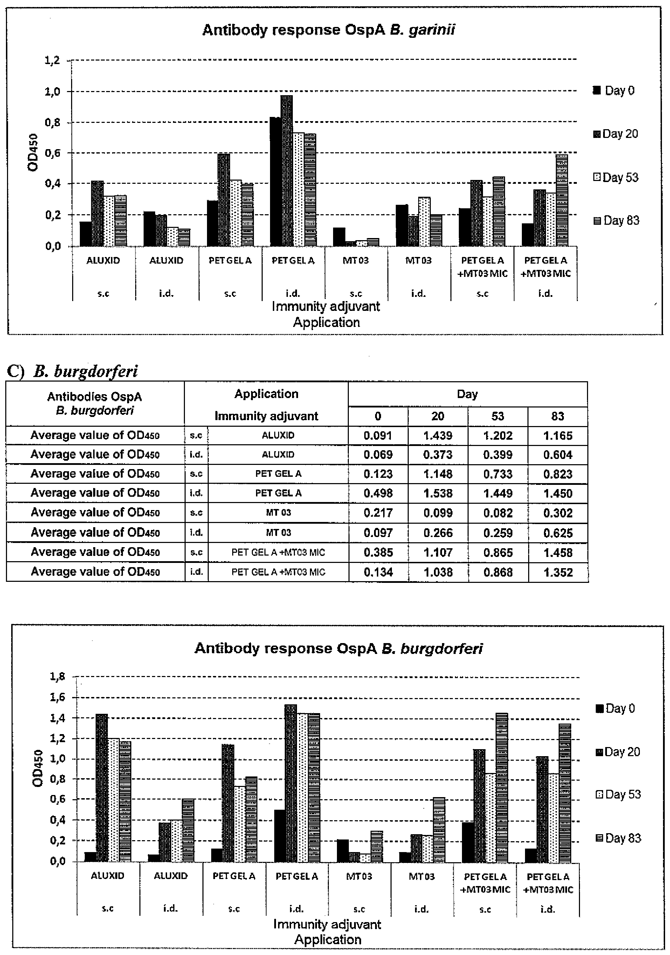

**Fig. 15** Determination of antibody response in dogs against OspA from *B. afzelii* (A), *B. garinii* (B) and *B. burgdorferi* (C) after the administration of the recombinant chimeric antigen polyOspA in combination with different adjuvants. Experimental vaccines were administered i.d. and s.c.; the antibody response was monitored for 83 days. Molecular adjuvant MT03 in combination with PET GEL A was administered in the form of micelles.

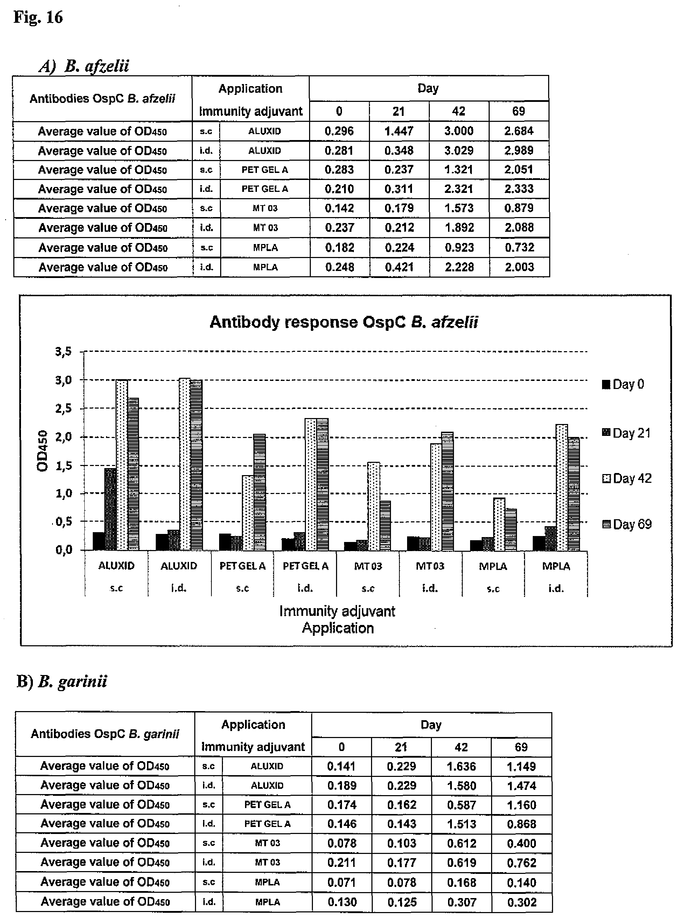

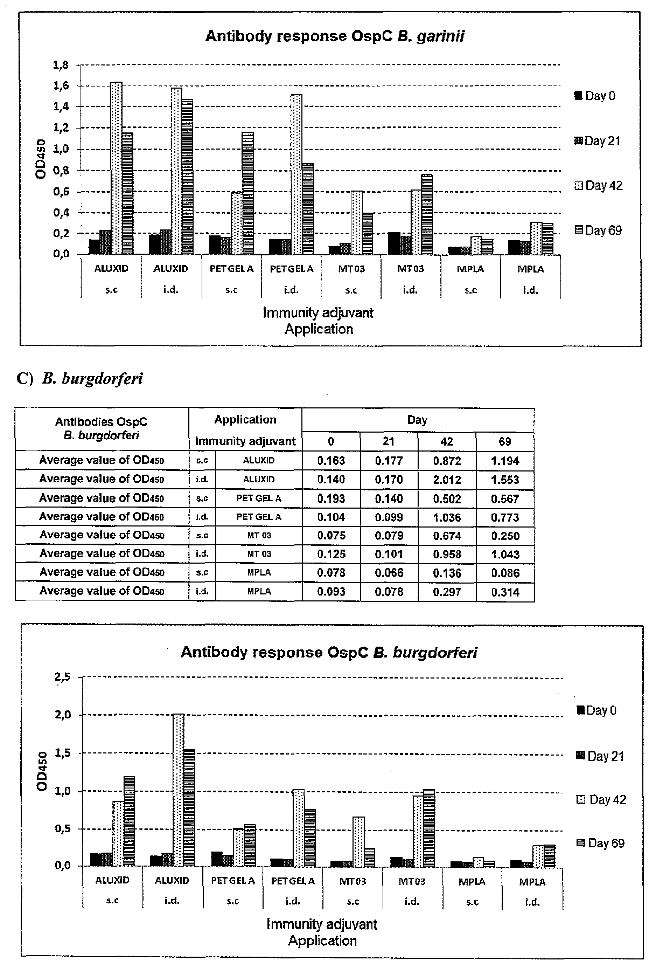

**Fig. 16****:** Determination of antibody response in dogs against OspC from *B. afzelii* (A), *B. garinii* (B) and *B. burgdorferi* (C) after the administration of the chimeric chimeric antigen polyOspC in combination with different adjuvants. Experimental vaccines were administered i.d. and s.c.; the antibody response was monitored for 83 days.

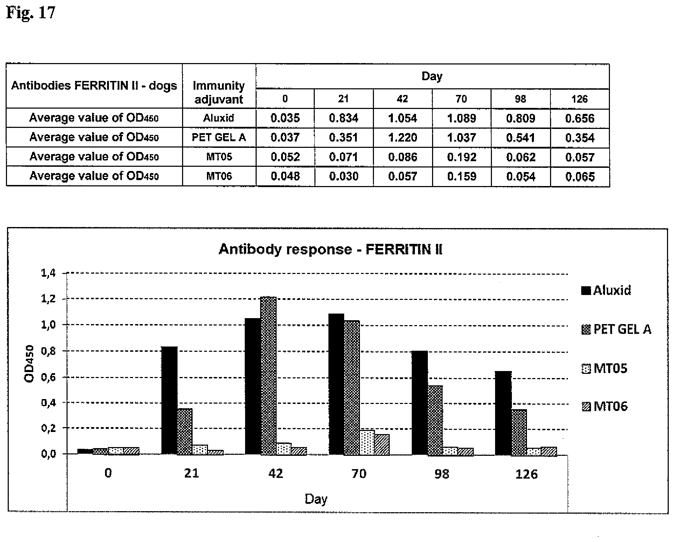

**Fig. 17** The induction of specific antibody response in dogs against recombinant Ferritin II formulated with different adjuvants.

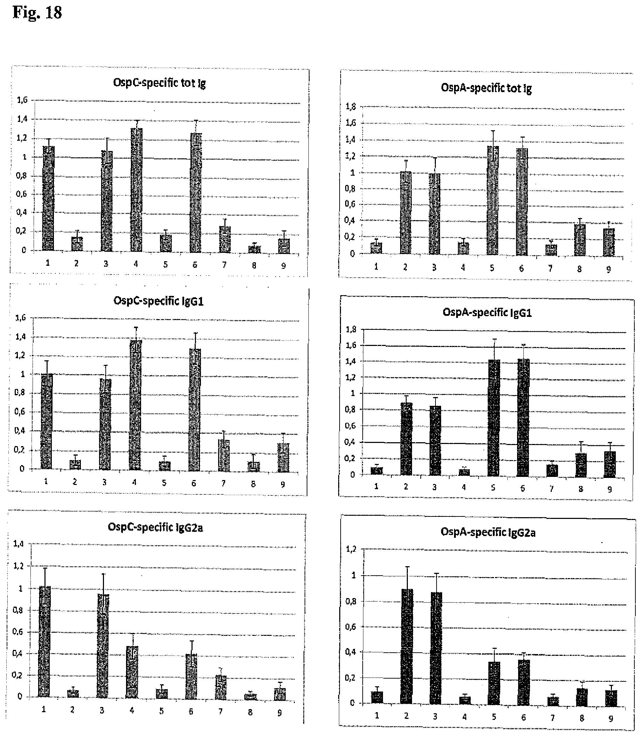

**Fig. 18** Antigen specific immune responses in total and selected isotypes to experimental vaccines containing recombinant antigens rOspA and rOspC.

### Examples of carrying out the Invention

### Example 1. Preparation of antigen: OspA polyepitope

Polyepitope OspA is produced by a recombinant technology from strains *Borrelia burgdorferi* sensu stricto, *Borrelia garinii* and *Borrelia afzelii* classified in groups PHei, PKa, *(Borrelia burgdorferi* s.s.), PBi, TN, N34, VS307, G25, PBr, T25 (*Borrelia garinii*) and PKo (*Borrelia afzelii*), characterised by their invasiveness and thus the ability to cause systemic infections. In the GenBank database, OspA proteins, fragments of which were assembled to the vaccination polyepitope OspA, are listed under the following designations with amino acid sequences:
X80257.1 - gi|258153: **OspA-PBi** from *B. garinii* (SEQ ID NO. 8) 
1 MKKYLLGIGL ILALIACKQN VSSLDEKNSV SVDLPGEMKV LVSKEKDKDG
51 KYSLMATVDK LELKGTSDKS NGSGTLEGEK SDKSKAKLTI SEDLSKTTFE
101 IFKEDGKTLV SKKVNSKDKS SIEEKFNAKG ELSEKTILRA NGTRLEYTEI
151 KSDGTGKAKE VLKDFALEGT LAADKTTLKV TEGTVVLSKH IPNSGEITVE
201 LNDSNSTQAT KKTGKWDSNT STLTISVNSK KTKNIVFTKE DTITVQKYDS
251 AGTNLEGNAV EIKTLDELKN ALK
S48322 - gi|258151: **OspA-PKo** from *B. afzelii* (SEQ ID NO. 9) 
1 MKKYLLGIGL ILALIACKQN VSSLDEKNSA SVDLPGEMKV LVSKEKDKDG
51 KYSLKATVDK IELKGTSDKD NGSGVLEGTK DDKSKAKLTI ADDLSKTTFE
101 LFKEDGKTLV SRKVSSKDKT STDEMFNEKG ELSAKTMTRE NGTKLEYTEM
151 KSDGTGKAKE VLKNFTLEGK VANDKVTLEV KEGTVTLSKE IAKSGEVTVA
201 LNDTNTTQAT KKTGAWDSKT STLTISVNSK KTTQLVFTKQ DTITVQKYDS
251 AGTNLEGTAV EIKTLDELKN ALK
X80251 - gi|984067: **OspA-PHei** from *B. burgdorferi* (SEQ ID NO. 10) 
1 MKKYLLGIGL ILALIACKQN VSSLDEKNSV SVDLPGGMKV LVSKEKDKDG
51 KYSLMATVEK IELKGTSDKN NGSGTLEGEK TDKSKVKLTI AEDLSKTTFE
101 IFKEDGKTLV SKKVTLKDKS STEEKFNEKG EISEKTIVRA NGTRLEYTDI
151 KSDKTGKAKE VLKDFTLEGT LAADGKTTLK VTEGTVTLSK NISKSGEITV
201 ALDDTDSSGN KKSGTWDSGT STLTISKNRT KTKQLVFTKE DTITVQNYDS
251 AGTNLEGKAV EITTLKELKN ALK
X80252 - gi|984085 **:OspA-TN, N34, VS307, G25** from *B. garinii,* **PWudll** from *B. burgdorferi* (SEQ ID NO. 11) 
1 MKKYLLGIGL ILALIACKQN VSSLDEKNSV SVDLPGGMTV LVSKEKDKDG
51 KYSLEATVDK LELKGTSDKN NGSGTLEGEK TDKSKVKLTI ADDLSQTKFE
101 IFKEDGKTLV SKKVTLKDKS STEEKFNEKG ETSEKTIVRA NGTRLEYTDI
151 KSDGSGKAKE VLKDFTLEGT LAADGKTTLK VTEGTVVLSK NILKSGEITV
201 ALDDSDTTQA TKKTGKWDSK TSTLTISVNS QKTKNLVFTK EDTITVQKYD
251 SAGTNLEGKA VEITTLKELK DALK
X80182 - gi|984069: **OspA-PKa** from *B. burgdorferi* (SEQ ID NO. 12) 
1 MKKYLLGIGL ILALIACKQN VSSLDEKNSV SVDLPGEMKV LVSKEKNKDG
51 KYDLIATVDK LELKGTSDKN NGSGVLEGVK ADKSKVKLTI SDDLGQTTLE
101 VFKEDGKTLV SKKVTSKDKS STEEKFNEKG EVSEKIITRA DGTRLEYTGI
151 KSDGSGKAKE VLKGYVLEGT LTAEKTTLVV KEGTVTLSKN ISKSGEVSVE
201 LNDTDSSAAT KKTAAWNSGT STLTITVNSK KTKDLVFTKE NTITVQQYDS
251 NGTKLEGSAV EITKLDEIKN ALK
X80256 - gi|984079: **OspA-PBr** from *B. garinii* (SEQ ID NO. 13) 
1 MKKYLLGIGL ILALIACKQN VSSLDEKNSV SVDLPGGMKV LVSKEKDKDG
51 KYSLMATVEK LELKGTSDKS NGSGVLEGEK ADKSKAKLTI SQDLNQTTFE
101 IFKEDGKTLV SRKVNSKDKS STEEKFNDKG KLSEKVVTRA NGTRLEYTEI
151 KNDGSGKAKE VLKGFALEGT LTDGGETKLT VTEGTVTLSK NISKSGEITV
201 ALNDTETTPA DKKTGEWKSD TSTLTISKNS QKPKQLVFTK ENTITVQNYN
251 RAGNALEGSP AEIKDLAELK AALK
X80254 - gi|984081: **OspA-T25** from *B. garinii* (SEQ ID NO. 14)

1 MKKYLLGIGL ILALIACKQN VSSLDEKNSV SVDLPGEMKV LVSKEKDKDG
51 KYSLEATVDK LELKGTSDKN NGSGVLEGVK AAKSKAKLTI ADDLSQTKFE
101 IFKEDGKTLV SKKVTLKDKS STEEKFNDKG KLSEKVVTRA NGTRLEYTEI
151 QNDGSGKAKE VLKSLTLEGT LTADGETKLT VEAGTVTLSK NISESGEITV
201 ELKDTETTPA DKKSGTWDSK TSTLTISKNS QKTKQLVFTK ENTITVQKYN
251 TAGTKLEGSP AEIKDLEALK AALK

For the construction of OspA polyepitope, segments corresponding to the C terminal barrel-like structure region consisting of β14 to β21 sheets of each of the aforementioned OspA proteins, see the following table. Polyepitope is terminated with C'-terminal α-helix. The chimeric OspA polyepitope does not contain sequences which were identified in previous studies as potentially cross-(auto)reactive with human LFA-1 protein (amino acid region 165-173). At N¹-terminus, OspA polyepitope further comprises three peptide segments: 1) hexa His-tag allowing protein purification using Ni-NTA affinity chromatography and preparation of metallochelating proteoliposomes, 2) a thrombin cleavage site allowing the removal of His-tag from the recombinant protein for the immunodetection ELISA applications determining the levels of antigen induced-antibodies in immunized subjects, 3) T7 epitope allowing easy detection of the recombinant protein during the biotechnological production and purification using anti-T7 antibody.

| Designation in GeneBank database | Sequence region in amino acids | Classification of OspA antigen group |
|---|---|---|
| X80257.1 - gi\|258153 | **178-258** | *B. garinii* **PBi** |
| S48322 - gi\|258151 | **178-258** | *B. afzelii* **PKo** |
| X80251 - gi\|984067 | **179-258** | *B. burgdorferi* **PHei** |
| X80252 - gi\|984085 | **178-259** | *B. garinii* **TN, N34, VS307, G25** *B. burgdorferi* **PWudll** |
| X80182 - gi\|984069 | **179-258** | *B. burgdorferi* **PKa** |
| X80256 - gi\|984079 | **179-259** | *B. garinii* **PBr** |
| X80254 - gi 984081 | **179-259** | *B. garinii* **T25** |

The resulting polyepitope OspA has the following amino acid sequence (SEQ ID NO. 15)

1 MGSSHHHHHH SSGLVPRGSH MASMTGGQQM GRGSLKVTEG TVVLSKHIPN
51 SGEITVELND SNSTQATKKT GKWDSNTSTL TISVNSKKTK NIVFTKEDTI
101 TVQKYDSAGT NLEGNLEVKE GTVTLSKEIA KSGEVTVALN DTNTTQATKK
151 TGAWDSKTST LTISVNSKKT TQLVFTKQDT ITVQKYDSAG TNLEGTLKVT
201 EGTVTLSKNI SKSGEITVAL DDTDSSGNKK SGTWDSGTST LTISKNRTKT
251 KQLVFTKEDT ITVQNYDSAG TNLEGKTLKV TEGTVVLSKN ILKSGEITVA
301 LDDSDTTQAT KKTGKWDSKT STLTISVNSQ KTKNLVFTKE DTITVQKYDS
351 AGTNLEGKLV VKEGTVTLSK NISKSGEVSV ELNDTDSSAA TKKTAAWNSG
401 TSTLTITVNS KKTKDLVFTK ENTITVQQYD SNGTKLEGSL TVTEGTVTLS
451 KNISKSGEIT VALNDTETTP ADKKTGEWKS DTSTLTISKN SQKPKQLVFT
501 KENTITVQNY NRAGNALEGS LTVEAGTVTL SKNISESGEI TVELKDTETT
551 PADKKSGTWD SKTSTLTISK NSQKTKQLVF TKENTITVQK YNTAGTKLEG
601 SAVEITKLDE IKNALKKDLE ALKAALK

A three-dimensional predictive model of the structure of this chimeric OspA polyepitope is shown in **Fig. 1****.** The recombinant chimeric fusion protein (polyOspA) composed of the immunodominant regions of the OspA proteins described in clinically most significant strains of Borrelia, was fused at the N' terminus with His-tag for metal-affinity purification. A gene construct for polyOspA was inserted into plasmid pET28 enabling the induced expression of the gene in a bacterial system after the addition of isopropyl-β-D-thiogalactoside. The protein was expressed in the bacterial strain *E. coli* BL21 (DE3) and purified from bacterial lysate using metal-affinity chromatography under native conditions, using the binding of His tag to Ni-NTA agarose. For the column washing and elution of the target protein, buffers containing imidazole as a competitor of the affinity binding were used. The outcome of the OspA polyepitope purification, using affinity chromatography with Ni-NTA agarose, is shown in **Fig. 2****.**

### Example 2. Preparation of antigen: OspC polyepitope

Polyepitope OspC is produced by a recombinant technology from strains *Borrelia burgdorferi* sensu stricto, *Borrelia garinii* and *Borrelia afzelii* classified in groups A, B, I and Kb (*Borrelia burgdorferi* s. s.), Pbi *(Borrelia garinii)* and PKo, *{Borrelia afzelii)* characterised by their invasiveness and thus the ability to cause systemic infections. In the GenBank database there are OspC proteins, fragments of which were assembled into the vaccine OspC polyepitope, listed under the following designation with the amino acid sequence:
NPJM7005 - gill 1497007: **OspC group A** (B31MI) from *B. burgdorferi* (SEQ ID NO. 1) 
1 MKKNTLSAIL MTLFLFISCN NSGKDGNTSA NSADESVKGP NLTEISKKIT
51 DSNAVLLAVK EVEALLSSID EIAAKAIGKK IHQNNGLDTE NNHNGSLLAG
101 AYAISTLIKQ KLDGLKNEGL KEKIDAAKKC SETFTNKLKE KHTDLGKEGV
151 TDADAKEAIL KTNGTKTKGA EELGKLFESV EVLSKAAKEM LANSVKELTS
201 PVVAESPKKP
EF053525 - gill 17574163: **OspC group B** (LDP73) from *B. burgdorferi* (SEQ ID NO. 2) 
1 NNSGKDGNTS ANSADESVKG PNLTEISKKI TDSNAVLLAV KEVEALLSSI
51 DELAKAIGKK IKNDGSLDNE ANRNESLLAG AYTISTLITQ KLSKLNGSEG
101 LKEKIAAAKK CSEEFSTKLK DNHAQLGIQG VTDENAKKAI LKANAAGKDK
151 GVEELEKLSG SLESLSKAAK EMLANSVKEL TSPVVAESPK KP
AF467874 - gi|23507072: **OspC group I** (B331) from *B. burgdorferi* (SEQ ID NO. 3) 
1 MKKNTLSAIL MTLFLFISCN NSGKDGNTSA NSADESVKGP NLTEISKKIT
51 ESNAVVLAVK EVETLLTSID ELAKAIGKKI KNDVSLDNEA DHNGSLISGA
101 YLISTLITKK ISAIKDSGEL KAEIEKAKKC SEEFTAKLKG EHTDLGKEGV
151 TDDNAKKAIL KTNNDKTKGA DELEKLFESV KNLSKAAKEM LTNSVKELTS
201 PVVAESPKKP
EF053517 - gill 17574147: **OspC group K** (LDP74) from *B. burgdorferi* (SEQ ID NO. 4) 
1 NNSGKDGNTS ANSADESVKG PNLTEISKKI TESNAVVLAV KEIETLLASI
51 DELATKAIGK KIQQNGGLAV EAGHNGTLLA GAYTISKLIT QKLDGLKNSE
101 KLKEKIENAK KCSEDFTKKL EGEHAQLGIE NVTDENAKKA ILITDAAKDK
151 GAAELEKLFK AVENLAKAAK EMLANSVKEL TSPIVAESPK KP
YP_063261 - gi|51038597: **OspC group PBi** from *B. garinii* (SEQ ID NO. 5) 
1 MKKNTLSAIL MTLFLFISCN NSGGDSASTN PDESAKGPNL TVISKKITDS
51 NAFLLAVKEV EALLSSIDEL SKAIGKKIKN DGTLDNEANR NESLIAGAYE
101 ISKLITQKLS VLNSEELKEK IKEAKDCSEK FTTKLKDSHA ELGIQSVQDD
151 NAKKAILKTH GTKDKGAKEL EELFKSLESL SKAAQAALTN SVKELTNPVV
201 AETPKKP
YP_709265 - gill 11074118: **OspC group PKo** from *B. afzelii* (SEQ ID NO. 6)

1 MKKNTLSAIL MTLFLFISCN NSGKGGDSAS TNPADESAKG PNLTEISKKI
51 TDSNAFVLAV KEVETLVLSI DELAKKAIGQ KIDNNNGLAA LNNQNGSLLA
101 GAYAISTLIT EKLSKLKNLE ELKTEIAKAK KCSEEFTNKL KSGHADLGKQ
151 DATDDHAKAA ILKTHATTDK GAKEFKDLFE SVEGLLKAAQ VALTNSVKEL
201 TSPVVAESPK KP

Segments corresponding to α3-L5-α4-L6-α5 regions of particular OspC proteins were used for the construction of OspC polyepitope, see the following table. The polyepitope is terminated with the C'-terminal segment derived from OspC PKo. At N¹-terminus, the protein comprises three peptide segments: 1) hexa His-tag allowing protein purification by means of Ni-NTA affinity chromatography and production of metallochelating proteoliposomes, 2) a thrombin cleavage site allowing the removal of His-tag from the recombinant protein for immunodetection (ELISA) applications determining the levels of antigen-induced specific antibodies in immunized subjects, 3) T7 epitope allowing easy detection of the recombinant protein during the biotechnological production and purification using anti-T7 antibody.

| Designation in GeneBank database | Selected sequence region - amino acid sequence | Classification of OspC antigen according to the group |
|---|---|---|
| NP_047005 - gi\| 11497007 | **128-196** | *B. burgdorferi* group **A** (B31MI) |
| EF053525 - gill 17574163 | **109-178** | *B. burgdorferi* group **B** (LDP73) |
| AF467874 - gi\|23507072 | **128-196** | *B. burgdorferi* group **I** (B331) |
| EF053517 - gill 17574147 | **110-178** | *B. burgdorferi* group **K** (LDP74) |
| YP 063261 - gi\|51038597 | **125-193** | *B. garinii* group **PBi** |
| YP_709265 - gi\|1 11074118 | **130-211** | *B. afzelii* group **Pko** |

The resulting OspC polyepitope has the following amino acid sequence (SEQ ID NO. 7):

1 MGSSHHHHHH SSGLVPRGSH MASMTGGQQM GRGSMKKCSE EFSTKLKDNH
51 AQLGIQGVTD ENAKKAILKA NAAGKDKGVE ELEKLSGSLE SLSKAAKEML
101 ANSVKKKCSE DFTKKLEGEH AQLGIEQVTD ENAKKAILIT DAAKDKGAAE
151 LEKLFKAVEN LAKAAKEMLA NSVKKKCSET FTNKLKEKHT DLGKEGVTDA
201 DAKEAILKTQ GTKTKGAEEL GKLFESVEVL SKAAKEMLAN SVKKKCSEEF
251 TAKLKGEHTD LGKEGVTDDN AKKAILKTNN DKTKGADELE KLFESVKQLS
301 KAAKEMLTNS VKKDCSEKFT TKLKDSHAEL GIQSVQDDNA KKAILKTHGT
351 KDKGAKELEE LFKSLESLSK AAQAALTNSV KKKCSEEFTN KLKSGHADLG
401 KQDATDDHAK AAILKTHATT DKGAKEFKDL FESVEGLLKA AQVALTNSVK
451 ELTSPVVAES PKKN

A 3D predictive model of this chimeric OspC polyepitope structure is shown in **Fig 3****.** The recombinant chimeric fusion protein (polyOspC) composed of the immunodominant regions of OspC proteins described for the clinically most important strains of Borrelia, fused with His-tag to the N¹ terminus for metal-affinity purification. A gene construct for polyOspC was inserted into plasmid pET28 enabling induced gene expression in the bacterial system after the addition of isopropyl-β-D-thiogalactoside. The protein was expressed in bacterial strain *E. coli* BL21 (DE3) and purified from a bacterial lysate by metal-affinity chromatography under native conditions using the binding of His-tag to Ni-NTA agarose. For washing the column and elution of the target protein, buffers containing imidazole as the binding affinity competitor were used. The outcome of the OspC polyepitope purification by affinity chromatography with Ni-NTA agarose is shown in **Fig. 4****.**

### Example 3: Characterization of antigens

### Antigen size and thermal stability

The hydrodynamic radius and thermal stability of the antigen was measured by the dynamic light scattering method (DLS; ZetaSizer ZS, Malvern). The temperature gradient was chosen in the range of 10-60°C, at 0.5°C/min. The hydrodynamic diameter was 5.3 nm and 6.1 nm for polyOspA and polyOspC antigen, respectively. The calculation was performed in the mode of the highest number of particles.

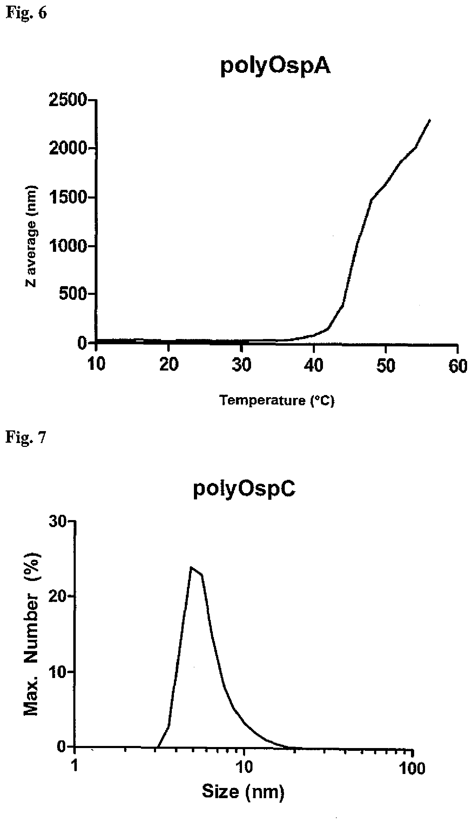

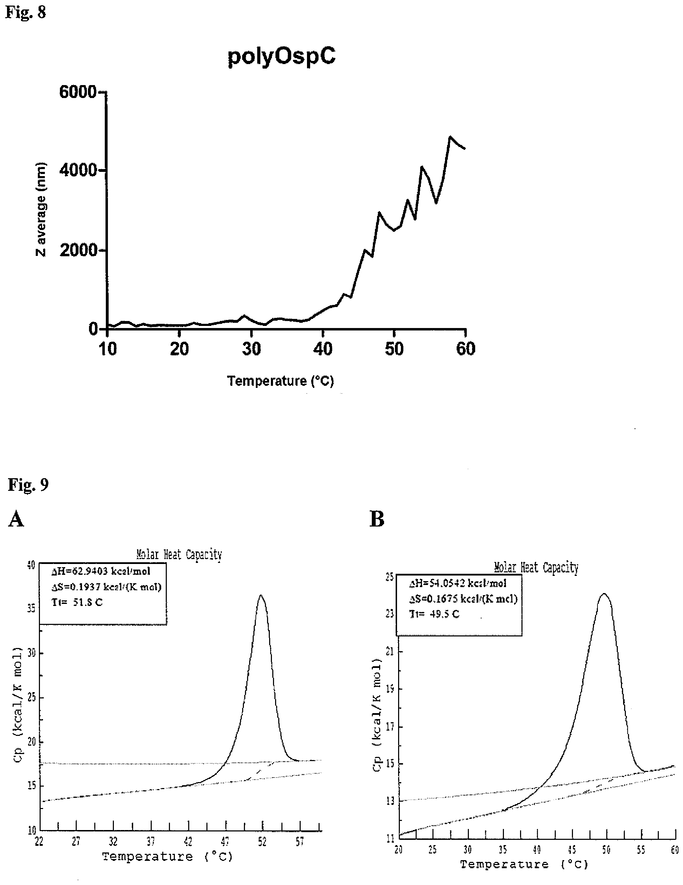

**Fig. 5** and **6** show size distribution and thermal stability of polyOspA. **Fig. 7** and **8** show size distribution and thermal stability of polyOspC.

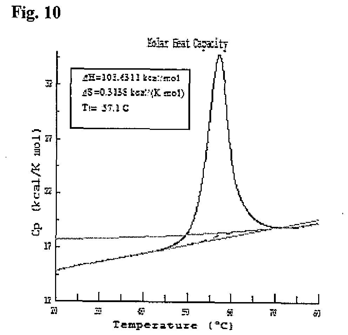

Thermal transitions and the influence of His-tag positions at the C- and N-terminus on thermal denaturation of polyOspC antigen, including thermodynamic parameters of thermal gradient are shown in **Fig. 9****.** The characteristics were obtained by differential scanning calorimetry (NanoDSC II, TA Instruments, USA) (protein concentration is 1 mg/ml in PBS). A) polyOspC His-tag at the N-terminus; B) polyOspC His-tag at the C-terminus. His-tag position at the N-terminus increases thermal stability by approximately 2°C compared to the C-terminus position. polyOspA was prepared with His-tag at the N-terminus and its thermal stability is higher than in polyOspA (**Fig. 10**).

### Example 4: Liposomal formulation of recombinant vaccines

Liposomal products were prepared by mixing polyOspA or polyOspC antigens (20 µg/dose) with liposomes (0.3 mg lipid/dose). As components for the production of liposomes, the following lipids from Avanti Polar Lipids (USA) were used: Egg phosphatidylcholine (EPC) (71 mol%), palmitoyl-oleoyl phosphatidyl glycerol (POPG) (19 mol%) and synthetic metal chelating lipid DOGS -NTA-Ni (1,2-di- (9Z-octadecenoyl) -sn-glycero-3 - [(N-(5-amino-1-carboxypenty)iminodiacetic acid) succinyl] (nickel salt)) (for antigen binding to liposome surface; 5 mol%). All lipids were purchased from Avanti Polar Lipids, USA. Liposomes were produced by hydration of the lipid film followed by extrusion through polycarbonate filters of 200 nm pore size (Millipore). As molecular adjuvant incorporated into the liposomes, monophosphoryl lipid A (MPLA) or the lipophilic derivative norAbuMDP (MT03) (structure and chemical name is shown in Supplement 1) at a dose of 5 mol% were used. This molar percentage corresponds to 32.6 µg MPLA or 21.4 µg MT03 per vaccination dose.

### Characterization of liposomes

The quality of liposomal products was assured by the techniques of DLS (ZetaSizer ZS, Malvern) and TEM (EM Philips 208 S, Morgagni software, FEI, Brno, CZ). The surface of liposomes was modified with antigen. The antigen was coupled by means of His-tag to the metallochelating lipid present in the liposome.

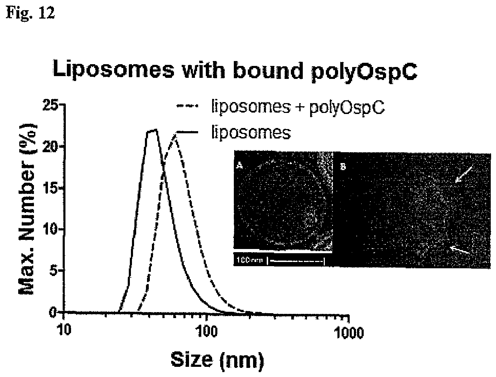

**Fig. 11** shows schematic structure of a liposomal vaccination particle. **Fig. 12** shows the detected changes in size distribution (DLS technique) which clearly confirm antigen binding to the metallochelating lipid in the liposome - the mean size of metal-chelating nanoliposomes is 48 nm and after OspC polyepitope binding, it changes to 67 nm. The TEM method showed unequivocally the metallochelating binding of antigens to liposomes (the inset of Fig. 13).

### Example 5. Immunization of mice

Mice were immunized with a recombinant OspA polyepitope (OspA Panel) or a recombinant OspC polyepitope (OspC Panel) in two doses administered i.d. at an interval of 14 days according to the following schemes.

### A) without adjuvant and liposomes

polyOspA (20 µg/dose) or free polyOspC protein (20 µg/dose)

### B) ALUXID

The products were prepared by mixing polyOspA or polyOspC antigen (50 µg/dose) with ALUXID (25 vol% of the final volume of the dose administered).

### C) PET GEL A

The products were prepared by mixing polyOspA or polyOspC antigen (50 µg/dose) and PET GEL A (Seppic) (5 vol% of the final volume of the dose administered).

### D) MT03-containing liposomes

The products were prepared by mixing polyOspA or polyOspC antigen (20 µg/dose) with liposomes (0.3 mg lipid/dose) containing MT03 (21.3 µg/dose) and incubated for at least 30 min at room temperature.

### E) MPLA-containing liposomes

The products were prepared by mixing polyOspA or polyOspC antigen (20 µg/dose) with liposomes (0.3 mg lipid/dose) containing MPLA (32.6 µg/dose) and incubated for 30 min at room temperature.

One month after the second dose, serum was collected and concentrations of OspA- or OspC-specific antibodies were determined by ELISA. The bottom of the wells was coated with recombinant proteins at a concentration of 100 ng per well. On the axis, the particular vaccine products are lettered in accordance with the text. In the case of immunization with a soluble recombinant OspA polyepitope, ELISA showed only the threshold antigen-specific responses for all IgG and IgM isotypes (IgG see **Fig. 13****,** IgM is not shown). Immunization with a soluble recombinant OspC polyepitope induced a stronger, but still a very weak immune (antibody) response. Conversely, immunization with recombinant OspA and OspC polyepitopes in the form of metallochelating liposomes with various adjuvants induced much higher antibody titres in different IgG and IgM isotypes (not shown for IgM). The key observation is the ability of the proteoliposome formulation of both OspA and OspC polyepitopes with MT03 and MPLA adjuvants to induce a dominant antigen-specific antibody response of IgG2a isotype which, in mice, is characterized by the ability to activate the classical complement pathway and mediate the opsonizing function, which are two mechanisms that in the case of Borrelia infections pertain among the primary host defence tools. In contrast, immunization with OspA and OspC proteoliposomes with aluminum adjuvant (Aluxid) and with GEL PET A induced predominantly a specific immune response of IgGl isotype ensuring that the antibodies had neutralizing activity, which in the case of Borrelia infections does not dominate in protective immunity.

The ability of the chimeric OspC polyepitope to elicit specific antibody responses was demonstrated after immunization of experimental mice. Antibodies react with different OspC antigens of various Borrelia species: *Borrelia. garinii, B. burgdorferi, B. afzelii* (band A) (**Fig. 14**). Conversely, immunization of mice with recombinant OspC from *B*. *burgdorferi* induced antibodies that did not react with all tested OspC from the main Borrelia species (band B). Serum from a non-immunized mouse did not react with any OspC antigen (band C).

### Preparation of micelles for muramyl dipeptide-based adjuvants

Lipophilic muramyl dipeptide (MDP) analogues are of amphiphilic nature and therefore are able to spontaneously form micelles. The micellar form of lipophilic MDP analogues can be used as an adjuvant formulation for recombinant vaccines, either alone or in combination with other substances exhibiting the adjuvant effects.

The lipophilic derivative MDP is dissolved in a minimum volume of ethanol or DMSO and the solution is rapidly diluted with either water or a suitable buffer solution or saline.

Example of the solution of a micellar form of MT03 at a concentration of 1 mg MT03/ml:

| Component | Amount |
|---|---|
| MT03 | 1 mg |
| Ethanol (96%) | 30 µl |
| 0.9% NaCl | 970 µl |

The solution prepared in this way shall be mixed with antigen solution and, if need be, another adjuvant (PET GEL Alum) so that the final concentration of the two components corresponds to the required immunization dose.

The size of MT03 micelles is expressed as a hydrodynamic diameter in nanometres and ranges between 8 and 10 nm. Critical micelle concentration of MT03 was determined to be 100 µg/ml at 25°C and 30 µg/ml at 37°C (DLS method).

### Combinations of lipophilic derivatives of MDP with other adjuvants

Micellar forms of lipophilic derivatives of MDP may be combined with other adjuvants, such as salts of aluminium or PET GEL A. A synergistic action of the two adjuvants is thus achieved.

### Preparation of PET GEL A formulation potentiated by micellar form MT03

The solution of MT03 micelles shall be prepared as described above. The solution PET GEL A shall be separately diluted so that after mixing with MT03 micelle solution the required concentrations of both adjuvants is achieved.

The solution prepared in this way shall be mixed with antigen solution so that the final concentration or the amount of all components corresponds to the required immunization dose. The molecular adjuvant MT03 does not cause aggregation by antigens or PET GEL particles (measured by DLS, data not shown)

PET GEL A formulation potentiated with the micellar form of MT03:

| **Component** | **Final amount/dose** |
|---|---|
| MT03 | 40.2 µg |
| GEL PET A | 10% solution |
| 0.9% NaCl | ad 0.1 or 1 ml |
| polyOspA antigen | 50 µg |

### Example 6: Monitoring of antibody responses against OspA in dogs after immunization with the chimeric protein polyOspA with adjuvants Aluxid and PET GEL A, liposomal MT03 and PET GEL A + micellar MT03

*Antigen:* A recombinant chimeric fusion protein (polyOspA) was used for immunization.

### Animals

The experiment included 20 dogs aged 11-12 months. The animals were allocated into ten groups of two each. Each animal was given the antigen or placebo with an appropriate immune adjuvant in three doses at the interval of three weeks. The dose of the antigen (recombinant chimeric protein OspA - polyOspA, polyOspA) was 50 µg per dose in all cases. The vaccination dose was 1.0 ml s.c. or 0.1 ml i.d. Placebo (= an appropriate immune response potentiating adjuvant) was administered to the animals subcutaneously at the same intervals and equal dose (1.0 ml).

### Adjuvants:

Aluxid - Aluminium Hydroxide Gel Adjuvant, Brenntag Biosector Denmark
PET GEL A - SEPPIC
Liposomal MT03
Micellar MT03 + PET GEL A

### Liposome formulation

Liposomal formulations (MT03 or MPLA) were prepared by mixing polyOspA antigens (50 µg/dose) with liposomes (0.82 mg lipid per dose). As components for liposome formulation, the following lipids were used: EPC (71 mol%), POPG (19 mol%) and synthetic metallochelating lipid DOGS-NTA-Ni (for antigen binding to liposome surface; 5 mol%). All lipids were purchased from Avanti Polar Lipids, USA. Liposomes were prepared by the lipid film hydration method followed by extrusion through polycarbonate filters of 200 nm pore size (Millipore). As a molecular adjuvant incorporated into liposomes, monophosphoryl lipid A (MPLA) or lipophilic derivative norAbuMDP (MT03) at 5 mol% were used. This molar percentage corresponds to 88 µg of MPLA or 40 µg MT03 per vaccination dose.

In the MT03 and MPLA control groups, only liposomes (0.82 mg/dose) were used; these contained 40 µg MT03 or 88 µg MPLA without added antigen.

### Formulation of vaccine doses:

### polyOspA + Aluxid

Antigen - 50 µg
Aluxid - 10 vol%
Sterile saline - ad 1.0 or 0.1 ml
The compounds were mixed and homogenized by agitation.

### polyOspA + PET GEL A

Antigen - 50 µg
PET GEL A - 10 vol%
Sterile saline - ad 1.0 or 0.1 ml
The compounds were mixed and homogenized by agitation.

### polyOspA + liposomal MT03

Antigen - 50 µg
Antigen binding to the immunity adjuvant was carried out as shown in Example 4.

### polyOspA + micellar MT03 + PET GEL A

Antigen - 50 µg
PET GEL A - 10 vol%
Micellar MT03 - according to the instructions (see Example 4)
Sterile saline - ad 1.0 or 0.1 ml
The compounds were mixed and homogenized by agitation.

### PLACEBO = Aluxid

Antigen - 0 µg
Aluxid - 10 vol%
Sterile saline - ad 1.0 or 0.1 ml
The compounds were mixed and homogenized (the vial content agitation)

### PLACEBO = PET GEL A

Antigen - 0 µg
PET GEL A - 10 vol%
Sterile saline - ad 1.0 or 0.1 ml
The compounds were mixed and homogenized (the vial content agitation)

### PLACEBO = MT 03

Antigen - 0 µg
Production - VRI

### PLACEBO = micellar MT03 + PET GEL A

Antigen - 0 µg
PET GEL A + micellar MT03 - 10 vol% (according to the instructions, see Example 4)
Sterile saline - ad 1.0 or 0.1 ml
The compounds were mixed and homogenized (the vial content agitation)

### Blood sampling

Blood was collected from all animals at day 0 (prior to primary vaccination), day 20 (prior to 1^{st}revaccination), day 53 (prior to 2^{nd} revaccination) and then at an interval of approximately once every 4 weeks until the end of the experiment (at least 12 months). After coagulation, serum was separated from the blood samples.

### Monitoring of animals during the experiment:

Animals were monitored on a daily basis for 14 days after each administration, and the occurrence of local and systemic post-vaccination reactions were assessed.

Neither local nor systemic post-vaccination reactions were observed in any of the animals. Body temperature was measured daily in all animals 3 days prior to each administration, on the day of the administration, 4 hours after the administration and then for 4 days after administration. During monitoring, no body temperature readings were found to be outside the physiological range.

### Experiment schedule

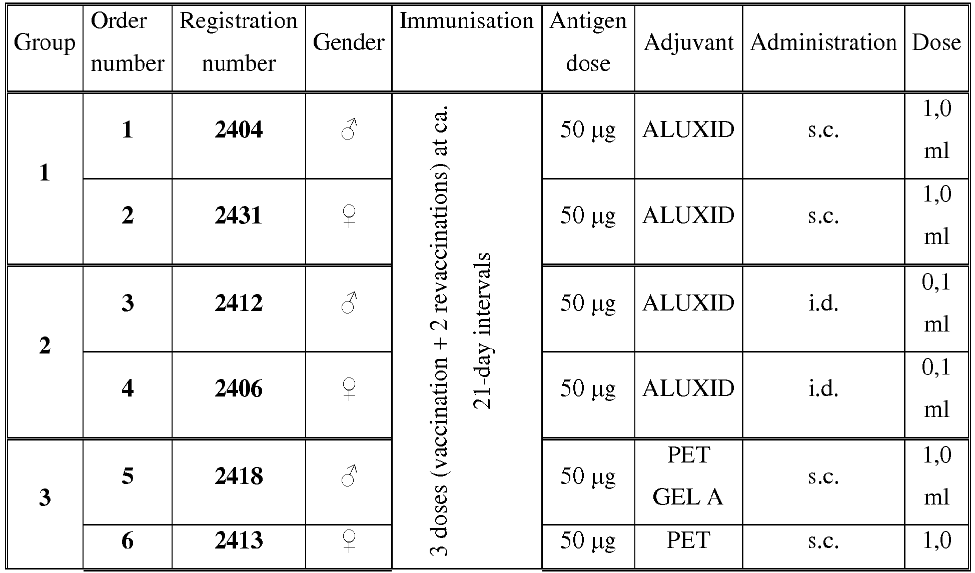

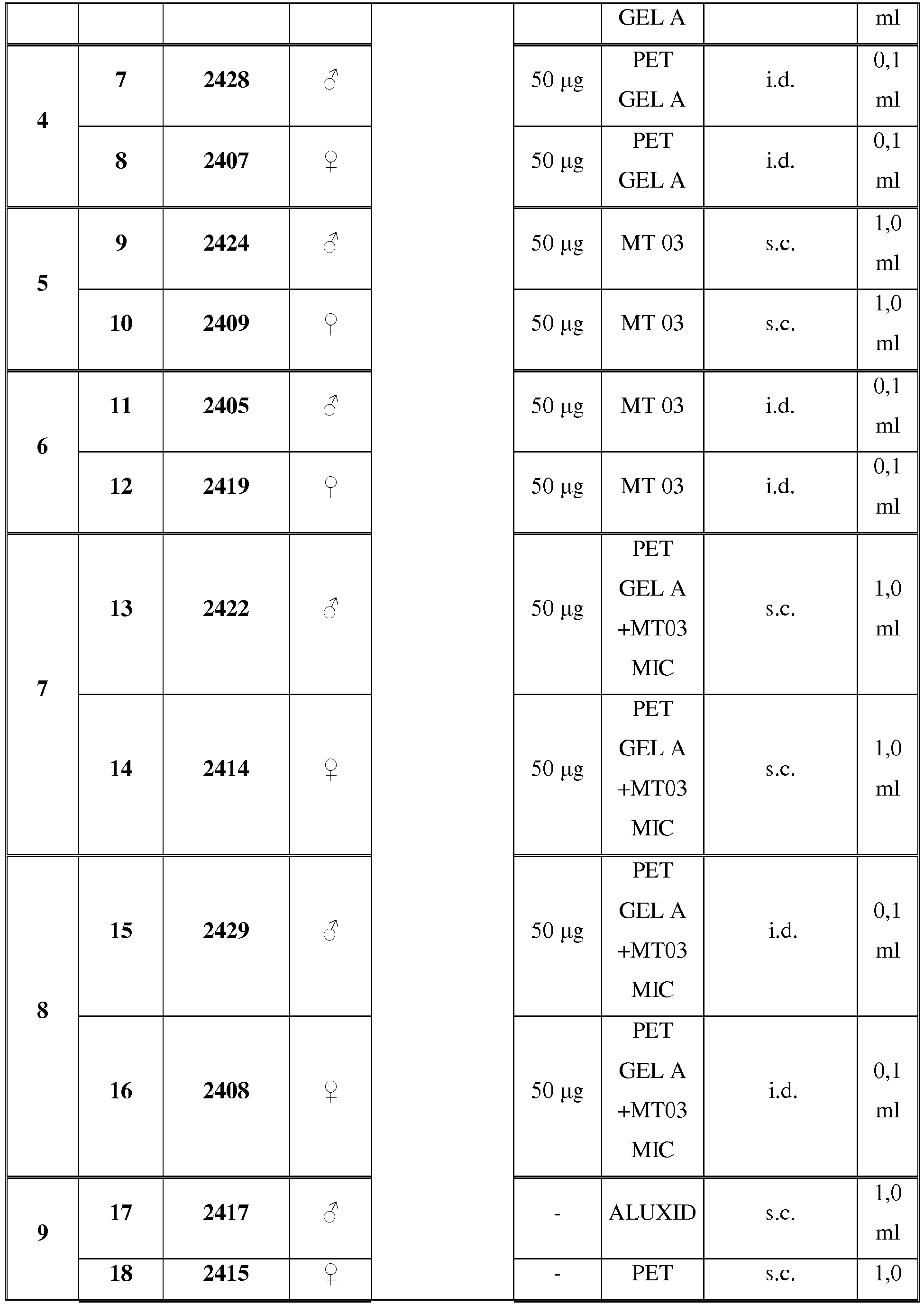

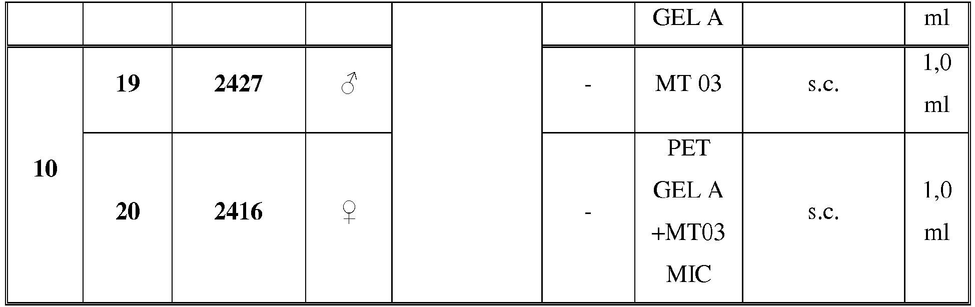

### Time schedule

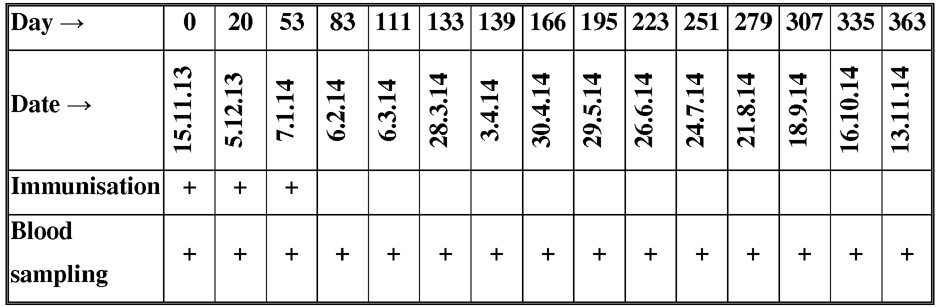

### Determination of the post-vaccination anti OspA antibodies in dog sera by ELISA

### Antigens used for microtiter plate coating

### • rOspA antigen from B. afzelii

The antigen is a recombinant protein isolated from the genetically modified strain of *E. coli* MSLB 7010 (*E. coli* BL21(DE3)/pDEST17).

### • rOspA antigen from B. garinii

The antigen is a recombinant protein isolated from the genetically modified strain of *E. coli* MSLB 7019 (*E. coli* BL21(DE3)/pCRT7/NT).

### • rOspA antigen from B. burgdorferi sensu stricto

The antigen is a recombinant protein isolated from the genetically modified strain of *E. coli* MSLB 7012 (*E. coli* BL21(DE3)/pDEST17).

### Production of antigens for microtiter plate coating

For microtiter plate coating, the aforementioned recombinant OspA proteins were used.

### Positive control sera

Positive control sera were produced by immunisation of dogs with inactivated strains of *B.* *afzelii, B. garinii* and *B. burgdorferi* sensu stricto with Freund's adjuvant. Negative control serum was prepared from a non-vaccinated dog.

### Evaluation of the antibody response after immunization of experimental animals on the basis of the OD₄₅₀ values obtained

**OD measurement: OD is measured at a wavelength of 450 nm against BLANK value.**

Evaluation: the average value of OD₄₅₀ of tested sample replicates is used. The test is evaluable if: CV of replicates is ≤15%

The examination includes 4 consecutive sampling occasions
1) initial (prior to vaccination)
2) 3 weeks after vaccination (prior to the 1st revaccination)
3) 53 days after the 1st revaccination (prior to the 2nd revaccination)
4) 4 weeks after the 2nd revaccination

In the diagrams, OD₄₅₀ values are compared with OD₄₅₀ values of the positive control and negative control sera.

The course of the immune response in dogs to different experimental vaccine formulations with polyOspA antigen is shown in **Fig. 15****.**

### Conclusion:

All adjuvants were able to induce the production of specific antibodies. Regarding the induction of antibody response against OspA from *B. afzelii,* OspA *B. garinii* and OspA *B. burgdorferi* sensu stricto after vaccination of dogs with chimeric OspA (polyOspA) protein, PET GEL A and PET GEL A in combination with micellar MT03 appear to be the best immune response adjuvants.

### Example 7: Monitoring of the antibody response against OspC in dogs after immunization with chimeric protein polyOspC with adjuvants Aluxid and PET GEL A and with liposomal adjuvants MT03 and MPLA

*Antigen:* Immunization was carried out with recombinant chimeric fusion protein (polyOspC).

### Animals

The experiment included 20 dogs aged 10-13 months. The animals were divided into ten groups of two each. Each animal was given the antigen or placebo with an appropriate immune adjuvant in three doses at the interval of three weeks. The dose of the antigen (recombinant chimeric OspC protein - polyOspC, polyOspC) was 50 µg per dose in all cases. The vaccination dose was 1.0 ml s.c. or 0.1 ml i.d. Placebo (=an appropriate immune response boosting adjuvant) was subcutaneously administered subcutaneously to animals at the same intervals and equal dose (1.0 ml).

### Adjuvants:

Aluxid - Aluminum Hydroxide Gel Adjuvant, Brenntag Biosector Denmark
PET GEL A - SEPPIC
Liposomal MT03
Liposomal MPLA

### Production of liposomes

Liposomal products (MT03 or MPLA) were prepared by mixing polyOspC antigens (50 µg/dose) with liposomes (0.82 mg lipid/dose) similarly to Example 6.

### Production of vaccination doses:

### polyOspC + Aluxid

Antigen - 50 µg
Aluxid - 10 vol%
Sterile saline - ad 1.0 or 0.1 ml
The compounds were mixed and homogenized by agitation.

### polyOspC + PET GEL A

Antigen - 50 µg
PET GEL A - 10 vol%
Sterile saline - ad 1.0 or 0.1 ml
The compounds were mixed and homogenized by agitation.

### polyOspC + liposomal MT03

Antigen - 50 µg Liposomes - 0.82 mg
Sterile saline - ad 1.0 or 0.1 ml
The solutions of antigen and liposomes were mixed and incubated at room temperature for at least 30 minutes.

### polyOspC + liposomal MPLA

Antigen - 50 µg
Liposomes - 0.82 mg
Sterile saline - ad 1.0 or 0.1 ml

The solutions of antigen and liposomes were mixed and incubated at room temperature for at least 30 minutes.

### PLACEBCO=Aluxid

Antigen - 0 µg
Aluxid - 10 vol%
Sterile saline - ad 1.0 or 0.1 ml
The compounds were mixed and homogenized (vial content agitation)

### PLACEBO=PET GEL A

Antigen - 0 µg
PET GEL A - 10 vol%
Sterile saline - ad 1.0 or 0.1 ml
The compounds were mixed and homogenized (vial content agitation)

### PLACEBO =MT 03

Antigen - 0 µg

### PLACEBO =MPLA

Antigen - 0 µg

### Blood sampling

Blood was collected from all animals at day 0 (prior to primary vaccination), day 21 (prior to 1^{st}revaccination), day 42 (prior to 2^{nd} revaccination) and then at an interval of approximately once every 4 weeks until the end of the experiment (at least 12 months). After coagulation, serum was separated from the blood samples.

### Monitoring of animals during the experiment:

Animals were monitored similarly as in Example 6.

### Experiment schedule:

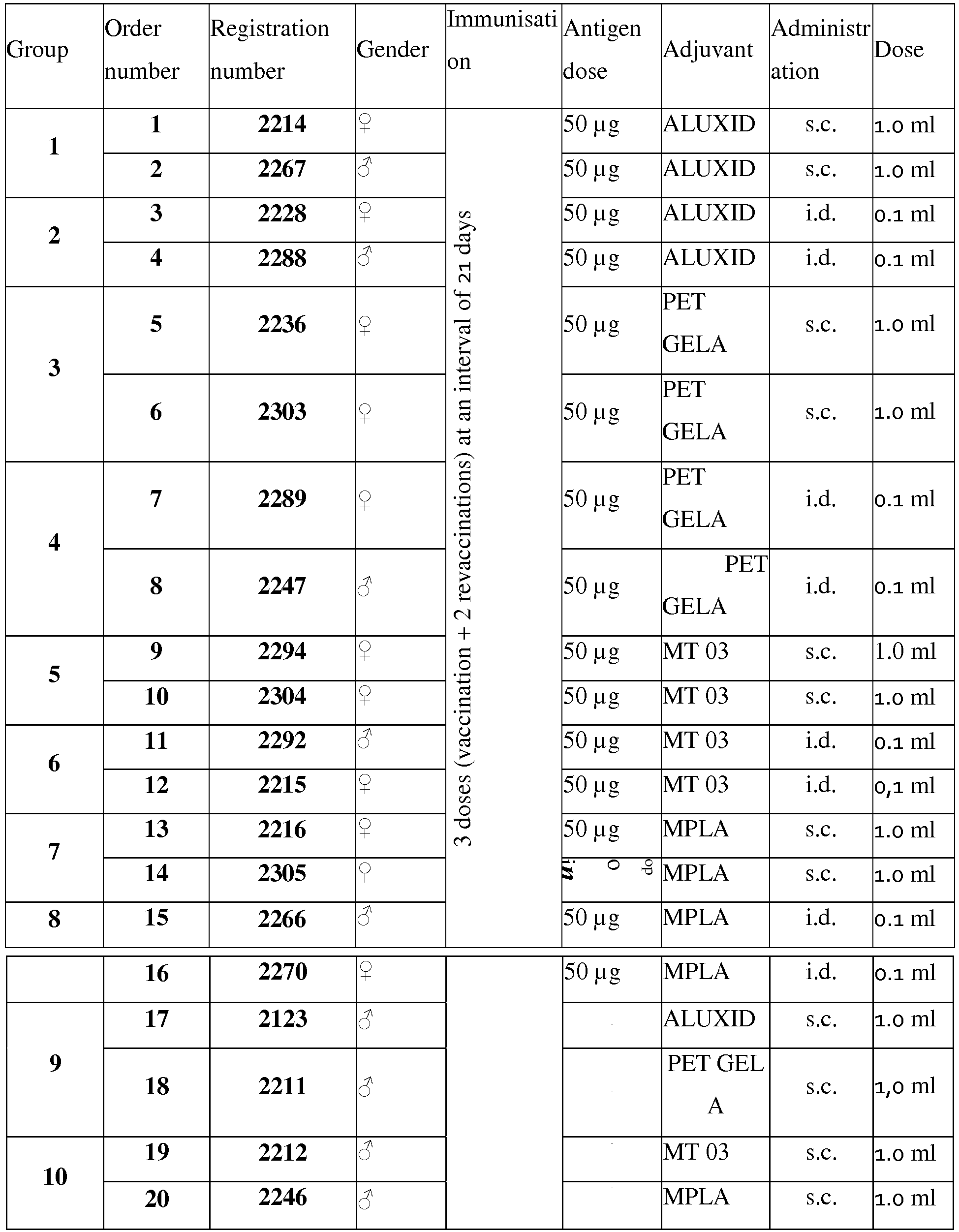

### Time schedule:

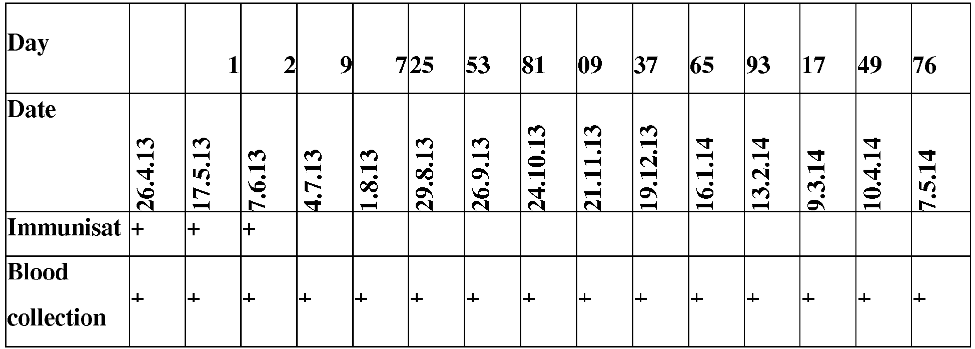

### Determination of the post-vaccination anti OspC antibodies in sera of dogs by ELISA

### Antigens used to coat the microtiter plates

### Antigen rOspC from B. afzelii

The antigen is a recombinant protein isolated from the genetically modified strain of *E. coli* MSLB 7002 (*E. coli* BL21(DE3)/pET28a).

### Antigen rOspA from B. garinii

The antigen is a recombinant protein isolated from the genetically modified strain of *E. coli* MSLB 7004 (*E. coli* BL21(DE3)/ pET28a).

### Antigen rOspA from B. burgdorferi sensu stricto

The antigen is a recombinant protein isolated from the genetically modified strain of *E. coli* MSLB 7003 (*E. coli* BL21(DE3)/ pET28a).

### Production of antigens for coating of the microtiter plate

For coating of the microtiter plate, the aforementioned recombinant rOspC proteins were used.

### Positive control sera

Positive control sera were prepared by immunisation of dogs using inactivated strains of *B. afzelii, B. garinii* and *B. burgdorferi* sensu stricto with Freund's adjuvant. Negative control serum was from a non-vaccinated dog.

### Evaluation of the antibody response after immunization of experimental animals on the basis of the OD₄₅₀ values obtained

**OD measurement: OD measurement is carried out at a wavelength of 450 nm against the value BLANK.**

Evaluation: The average value of OD₄₅₀ of replicates of a tested sample is used for the evaluation. The test is evaluable if: replicate CV ≤15%

The examination includes 4 successive sampling occasions
5) initial (prior to vaccination)
6) 3 weeks after vaccination (prior to the 1st revaccination)
7) 3 weeks after the 1^{st} revaccination (prior to the 2^{nd} revaccination)
8) 4 weeks after the 2^{nd} revaccination

In the diagrams, OD₄₅₀ values are compared with OD₄₅₀ values of the positive control and negative control sera.

The course of the immune response of dogs to different experimental formulations of vaccines with polyOspA antigen are shown in **Fig. 16****.**

### Conclusion:

The evaluation of antigen-specific antibody response induced by immunization of dogs with recombinant polyepitope OspC investigated two factors: A) cross-reactivity of canine hyperimmune sera with OspC antigens from various Borrelia species, B) the ability of the particular used adjuvant to induce OspC-specific serum antibodies of different IgG isotypes in dogs. As an example, we show different canine IgG isotype responses (after immunization with OspC polyepitope with different adjuvants) with OspC polyepitope bound to the bottom of the wells of ELISA panels, and with purified recombinant OspC from B. *afzelii.*

All used adjuvants tested were capable of inducing the formation of specific antibodies able to recognize all three types of OspC. Regarding the induction of antibody responses against OspC from *B. afzelii,* OspC from *B. garinii* and OspC from *B. burgdorferi* sensu stricto after vaccination of dogs with recombinant chimeric OspC protein (polyOspC) Aluxid (2% aluminum hydroxide gel) appears to be the best adjuvant enhancing the immune response.

### Example 8: Antibody response against the recombinant antigen rFerritin II. Monitoring of antibody response to Ferritin II in dogs after the administration of the antigen formulated with different adjuvants

### Antigen:

The recombinant protein FERRITIN II was used.

Ferritin II is an identified and characterized protein secreted into the tick plasma. Nucleotide sequences of Ferritin II gene were determined and their primary structure was defined *(*Kopacek et al. 2003. Insect Biochemistry and Molecular Biology 33, 103-113*).* Ferritin II in the tick body functions as a protective protein which binds and maintains iron ions released from hemoglobin and also substantially eliminates the oxidative stress caused by a high concentration of iron ions. Ferritin is responsible for blocking the toxicity of iron ions taken from the host blood (Galay et al. 2014 PLOS ONE 9, e90661)*.* If we manage to block the function of Ferritin, the attached tick will not be able to parasitize on the host because its intoxication with the non-heme bound iron ions will occur. By preventing its long-lasting feeding on the host the transmission of tick-borne pathogens can be blocked *(*Hajdusek et al. 2010 Vaccine 28, 2993- 2998*).* Blocking of Ferritin II can be achieved by the reaction with a specific antibody, which leads to the formation of an immune complex. Specific antibodies occur in the body after immunization with antigen.

Recombinant Ferritin II or its fragment produced by both prokaryotic *(E. coli)* and eukaryotic *(Pichia pastoris)* expression systems can be used as a suitable antigen for immunization.

### Animals

The experiment included 10 non-vaccinated dogs aged 3-4 months. The animals were divided into 4 groups of two or three each. Each animal was injected with the antigen containing an appropriate immunity potentiating adjuvant in three doses at an interval of three weeks. The antigen dosage (FERRITIN II) was uniform, namely 50 µg per dose. The vaccination dose was 1.0 ml s.c.

### Adjuvants:

Aluxid - Aluminium Hydroxide Gel Adjuvant, Brenntag Biosector Denmark
PET GEL A - (SEPPIC)
liposomal MT05
liposomal MT06

### Production of vaccination doses:

### FERRITIN II + Aluxid

Antigen - 50 µg
Aluxid - 10 vol%
Sterile saline - ad 1.0 ml
The components were mixed and homogenized by agitation.

### FERRITIN II + PET GEL A

Antigen - 50 µg
PET GEL A - 10 vol%
Sterile saline - ad 1.0 ml
The components were mixed and homogenized by agitation.

### FERRITIN II + MT 05

Antigen - 50 µg
liposomal MT05 - 0.88 mg
Sterile saline - ad 1.0 ml
The components were mixed and homogenized by agitation.

### FERRITIN II + MT 06

Antigen - 50 µg
liposomal MT06 - 0.88 mg
Sterile saline - ad 1.0 ml
The components were mixed and homogenized by agitation.

### Preparation of liposomes

Liposomal formulations (MT05 or MT06) were prepared by mixing the antigen FERRITIN II (20 µg/dose) with liposomes (0.88 mg lipid/dose). As components for the formation of liposomes, the following lipids were used: EPC (71 mol%), POPG (19 mol%) and the synthetic metallochelating lipid DOGS-NTA-Ni (for antigen binding to liposome surface, 5 mol.%). All lipids were purchased from Avanti Polar Lipids, USA. Liposomes were prepared by lipid film hydration method followed by extrusion through polycarbonate filters of 200 nm pore size (Millipore). As a molecular adjuvant incorporated into liposomes, lipophilic derivatives norAbuMDP (MT05 or MT06) at the dose of 5 mol% were used. This molar percentage corresponds to 56.4 nmol of the substance per vaccination dose (equivalent to 50 µg MT05 or 65 µg MT06).

### Antigen used for microtiter plate coating

Recombinant tick FERRITIN II (Institute of Parasitology BC, ACSR, Ceske Budejovice)

### Blood sampling

Blood was collected from all animals at day 0 (prior to primary vaccination), day 21 (prior to 1^{st}revaccination), day 42 (prior to 2^{nd} revaccination) and then at an interval of approximately once every 4 weeks until the end of the experiment (at least 12 months). After coagulation, serum was separated from the blood samples.

### Monitoring of animals during the experiment:

Animals were monitored on a daily basis for 14 days after each administration and the occurrence of local and systemic post-vaccination reactions was evaluated.
No local or systemic post-vaccination reactions were not observed in any of the animals.
Body temperature was measured daily in all animals 3 days prior to each administration, on the day of the administration, 4 hours after the administration and then for the period of 4 days after the administration. During monitoring, no body temperature readings were found to be outside the physiological range.

### Experiment schedule:

| Group | Order number | Registrat ion number | Gender | Vaccination 1.0 ml s.c. | | Revaccination 1.0 ml s.c. | | 2^{nd} revaccination 1.0 ml s.c. | |
|---|---|---|---|---|---|---|---|---|---|
| | | | | Antigen dose | Adjuva ant | Antigen dose | Adjuv ant | Antigen dose | Adjuv ant |
| 1 | 1 | ^{r} 2003 | ♀ | 50 µg | Aluxid | 50 µg | Aluxid | 50 µg | Aluxid |
| | 2 | 2004 | ♀ | 50 µg | Aluxid | 50 µg | Aluxid | 50 µg | Aluxid |
| 2 | 3 | 2012 | ♂ | 50 µg | PET GELA | 50 µg | PET GELA | 50 µg | PET GELA |
| | 4 | 2013 | ♂ | 50 µg | PET GELA | 50 µg | PET GELA | 50 µg | PET GELA |
| 3 | 5 | 2032 | ♀ | 50 µg | MT05 | 50 µg | MT05 | 50 µg | MT05 |
| | 6 | 2037 | ♂ | 50 µg | MT05 | 50 µg | MT05 | 50 µg | MT05 |
| | 7 | 2043 | ♂ | 50 µg | MT05 | 50 µg | MT05 | 50 µg | MT05 |
| 4 | 8 | 2053 | ♂ | 50 µg | MT06 | 50 µg | MT06 | 50 µg | MT06 |
| | 9 | 2054 | ♂ | 50 µg | MT06 | 50 µg | MT06 | 50 µg | MT06 |
| | 10 | 2056 | ♂ | 50 µg | MT06 | 50 µg | MT06 | 50 µg | MT06 |

### Time schedule:

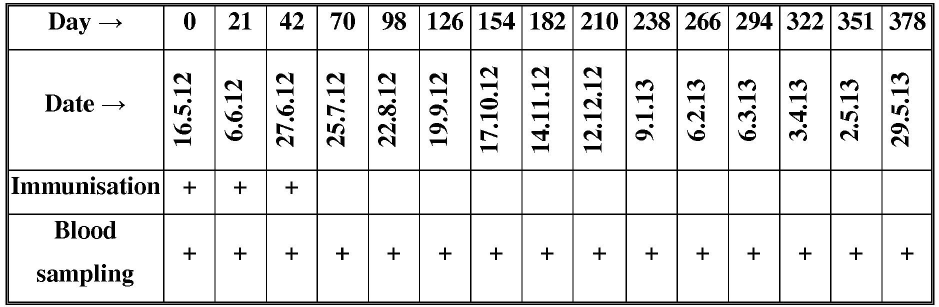

### ELISA detection of post-vaccination anti-FER II antibodies in dog sera Antigens used for microtiter plate coating

Recombinant FERRITIN II (Institute of Parasitology BC, ASCR, Ceske Budejovice).

Recombinant protein is stored frozen at -80° C.

### Substance

Control positive serum FERRITIN II.

Negative control serum.

Positive control sera were prepared by the immunization of dogs with recombinant FER II including Freund's adjuvant. Negative control serum was from a non-vaccinated dog.

### ELISA procedure

ELISA panels were coated with 100 µl of recombinant FERRITIN II.

### Evaluation

The average OD_{45°} value of replicates of a tested sample is used for the evaluation.
The test is evaluable if: CV of replicates ≤ 15%
The examination includes 15 consecutive samplings
OD_{45°} values in the diagrams are compared with the OD_{45°} values of the positive control and negative control serum.
Monitoring of antibody response in dogs to FER II after subcutaneous administration is shown in **Fig. 17**
rFerritin II formulation with various adjuvants
FERRITIN II + Aluxid
FERRITIN II + PET GEL A
FERRITIN II + MT05
FERRITIN II + MT06

### Conclusion:

The used adjuvants are capable of inducing a specific immune antibody response. Regarding the induction of antibody response to FER II after immunization of dogs, Aluxid (2% aluminum hydroxide gel) and PET GEL A appear to be the best adjuvants enhancing the immune response.

### Example 9. Immune response to a recombinant vaccine containing recombinant chimeric polyepitope antigens rOspC and OspA

Female BALB/c mice were immunised according to the following schedule:
1. Liposomes with bound rpolyOspC + MT 03
2. Liposomes with bound rOspA + MT 03
3. Liposomes with bound rOspA + MT 03 + Liposomes with bound rpolyOspC + MT 03
4. rpolyOspC + Alum
5. rOspA + Alum
6. rpolyOspC + rOspA + Alum
7. rpolyOspC
8. rOspA
9. rpolyOspC + rOspA

Experimental vaccines were prepared according to Examples 4 and 5. Immune responses to experimental vaccines are shown in **Figure 18****.**

### Conclusion:

The highest titre of total antibodies (tot Ig) was induced by immunization with Osp antigens in combination with alum adjuvant. Regarding the IgG1 isotype antibodies, which in the mouse are represented by the Th2 isotype, their highest levels were also induced by immunization with Osp antigens in combination with alum adjuvant. In contrast, immunization with Osp antigens on liposomes with MT03 adjuvant, elicited the highest titres of specific antibodies of IgG2a isotype, corresponding to Th1 type in the mouse. Immunization with various Osp antigens and their combinations did not lead to suppression of antibodies specific to different antigens.

## Claims

1. A vaccine suitable for treatment and prevention of Lyme borreliosis for human and veterinary use, **characterized in that** it contains
- chimeric polyepitope recombinant antigen having a sequence having at least 95% identity with the sequence (SEQ ID NO. 7) 
1 MGSSHHHHHH SSGLVPRGSH MASMTGGQQM GRGSMKKCSE EFSTKLKDNH
51 AQLGIQGVTD ENAKKAILKA NAAGKDKGVE ELEKLSGSLE SLSKAAKEML
101 ANSVKKKCSE DFTKKLEGEH AQLGIEQVTD ENAKKAILIT DAAKDKGAAE
151 LEKLFKAVEN LAKAAKEMLA NSVKKKCSET FTNKLKEKHT DLGKEGVTDA
201 DAKEAILKTQ GTKTKGAEEL GKLFESVEVL SKAAKEMLAN SVKKKCSEEF
251 TAKLKGEHTD LGKEGVTDDN AKKAILKTNN DKTKGADELE KLFESVKQLS
301 KAAKEMLTNS VKKDCSEKFT TKLKDSHAEL GIQSVQDDNA KKAILKTHGT
351 KDKGAKELEE LFKSLESLSK AAQAALTNSV KKKCSEEFTN KLKSGHADLG
401 KQDATDDHAK AAILKTHATT DKGAKEFKDL FESVEGLLKA AQVALTNSVK
451 ELTSPVVAES PKKN;
- chimeric polyepitope recombinant antigen having a sequence having at least 95% identity with the sequence (SEQ ID NO. 15) 
1 MGSSHHHHHH SSGLVPRGSH MASMTGGQQM GRGSLKVTEG TVVLSKHIPN
51 SGEITVELND SNSTQATKKT GKWDSNTSTL TISVNSKKTK NIVFTKEDTI
101 TVQKYDSAGT NLEGNLEVKE GTVTLSKEIA KSGEVTVALN DTNTTQATKK
151 TGAWDSKTST LTISVNSKKT TQLVFTKQDT ITVQKYDSAG TNLEGTLKVT
201 EGTVTLSKNI SKSGEITVAL DDTDSSGNKK SGTWDSGTST LTISKNRTKT
251 KQLVFTKEDT ITVQNYDSAG TNLEGKTLKV TEGTVVLSKN ILKSGEITVA
301 LDDSDTTQAT KKTGKWDSKT STLTISVNSQ KTKNLVFTKE DTITVQKYDS
351 AGTNLEGKLV VKEGTVTLSK NISKSGEVSV ELNDTDSSAA TKKTAAWNSG
401 TSTLTITVNS KKTKDLVFTK ENTITVQQYD SNGTKLEGSL TVTEGTVTLS
451 KNISKSGEIT VALNDTETTP ADKKTGEWKS DTSTLTISKN SQKPKQLVFT
501 KENTITVQNY NRAGNALEGS LTVEAGTVTL SKNISESGEI TVELKDTETT
551 PADKKSGTWD SKTSTLTISK NSQKTKQLVF TKENTITVQK YNTAGTKLEG
601 SAVEITKLDE IKNALKKDLE ALKAALK
- and at least one adjuvant and/or Ferritin II.

2. The vaccine according to claim 1, **characterized in that** the polyepitope antigens and/or adjuvant and/or ferritin II are bound on micro- or nano-particulate carriers containing a metallochelating component for binding said components.

3. The vaccine according to any one of the preceding claims, **characterized in that** the adjuvant is selected from the group comprising aluminum phosphate, aluminum hydroxide, potassium alum, and a molecular adjuvant based on normuramyl glycopeptides or their glycosylated forms, and an adjuvant on the basis of dispersion of particles prepared from biocompatible polymers.

4. The vaccine according to claim 3, wherein the adjuvant is selected from the group comprising aluminum phosphate, aluminum hydroxide, potassium alum, preferably the adjuvant is aluminium hydroxide.

5. The vaccine according to any one of the preceding claims for use in a method of prevention of Lyme borreliosis in veterinary and/or human medicine.

## Patentansprüche

1. Ein Impfstoff, der zur Behandlung und Vorbeugung von Lyme-Borreliose für den menschlichen und veterinärmedizinischen Gebrauch geeignet ist, und sich **dadurch gekennzeichnet, dass** er enthält
- rekombinantes chimäres Polyepitop-Antigen mit einer Sequenz mit mindestens 95% Identität mit der Sequenz (SEQ ID NO. 7) 
1 MGSSHHHHHH SSGLVPRGSH MASMTGGQQM GRGSMKKCSE EFSTKLKDNH
51 AQLGIQGVTD ENAKKAILKA NAAGKDKGVE ELEKLSGSLE SLSKAAKEML
101 ANSVKKKCSE DFTKKLEGEH AQLGIEQVTD ENAKKAILIT DAAKDKGAAE
151 LEKLFKAVEN LAKAAKEMLA NSVKKKCSET FTNKLKEKHT DLGKEGVTDA
201 DAKEAILKTQ GTKTKGAEEL GKLFESVEVL SKAAKEMLAN SVKKKCSEEF
251 TAKLKGEHTD LGKEGVTDDN AKKAILKTNN DKTKGADELE KLFESVKQLS
301 KAAKEMLTNS VKKDCSEKFT TKLKDSHAEL GIQSVQDDNA KKAILKTHGT
351 KDKGAKELEE LFKSLESLSK AAQAALTNSV KKKCSEEFTN KLKSGHADLG
401 KQDATDDHAK AAILKTHATT DKGAKEFKDL FESVEGLLKA AQVALTNSVK
451 ELTSPVVAES PKKN;
- rekombinantes chimäres Polyepitop-Antigen mit einer Sequenz mit mindestens 95% Identität mit der Sequenz (SEQ ID NO. 15) 
1 MGSSHHHHHH SSGLVPRGSH MASMTGGQQM GRGSLKVTEG TVVLSKHIPN
51 SGEITVELND SNSTQATKKT GKWDSNTSTL TISVNSKKTK NIVFTKEDTI
101 TVQKYDSAGT NLEGNLEVKE GTVTLSKEIA KSGEVTVALN DTNTTQATKK
151 TGAWDSKTST LTISVNSKKT TQLVFTKQDT ITVQKYDSAG TNLEGTLKVT
201 EGTVTLSKNI SKSGEITVAL DDTDSSGNKK SGTWDSGTST LTISKNRTKT
251 KQLVFTKEDT ITVQNYDSAG TNLEGKTLKV TEGTVVLSKN ILKSGEITVA
301 LDDSDTTQAT KKTGKWDSKT STLTISVNSQ KTKNLVFTKE DTITVQKYDS
351 AGTNLEGKLV VKEGTVTLSK NISKSGEVSV ELNDTDSSAA TKKTAAWNSG
401 TSTLTITVNS KKTKDLVFTK ENTITVQQYD SNGTKLEGSL TVTEGTVTLS
451 KNISKSGEIT VALNDTETTP ADKKTGEWKS DTSTLTISKN SQKPKQLVFT
501 KENTITVQNY NRAGNALEGS LTVEAGTVTL SKNISESGEI TVELKDTETT
551 PADKKSGTWD SKTSTLTISK NSQKTKQLVF TKENTITVQK YNTAGTKLEG
601 SAVEITKLDE IKNALKKDLE ALKAALK
- und mindestens ein Adjuvans und/oder Ferritin II.

2. Impfstoff nach Anspruch 1, **dadurch gekennzeichnet, dass** die Polyepitop-Antigene und/oder das Adjuvans und/oder Ferritin II an mikro- oder nanopartikuläre Träger gebunden sind, die eine metallochelierende Komponente zur Bindung der Komponenten enthalten.

3. Impfstoff nach einem der vorhergehenden Ansprüche, **dadurch gekennzeichnet, dass** das Adjuvans ausgewählt ist aus der Gruppe bestehend aus Aluminiumphosphat, Aluminiumhydroxid, Kaliumalum, einem molekularen Adjuvans auf Basis von Normuramylglycopeptiden oder deren glycosylierten Formen und einem Adjuvans auf die Basis der Dispersion von Partikeln, die aus biokompatiblen Polymeren hergestellt wurden.

4. Impfstoff nach Anspruch 3, wobei das Adjuvans ausgewählt ist aus der Gruppe umfassend Aluminiumphosphat, Aluminiumhydroxid, Kaliumalum, vorzugsweise ist das Adjuvans Aluminiumhydroxid.

5. Impfstoff nach einem der vorhergehenden Ansprüche zur Verwendung in einem Verfahren zur Vorbeugung von Lyme-Borreliose in der Veterinär- und/oder Humanmedizin.

## Revendications

1. Vaccin utilisable au traitement et à la prévention de la borréliose de Lyme à usage humain et vétérinaire, **caractérisé en ce qu'**il contient
- antigène recombinant polyépitopique chimérique ayant une séquence ayant au moins 95% d'identité avec la séquence (SEQ ID NO. 7) 
1 MGSSHHHHHH SSGLVPRGSH MASMTGGQQM GRGSMKKCSE EFSTKLKDNH
51 AQLGIQGVTD ENAKKAILKA NAAGKDKGVE ELEKLSGSLE SLSKAAKEML
101 ANSVKKKCSE DFTKKLEGEH AQLGIEQVTD ENAKKAILIT DAAKDKGAAE
151 LEKLFKAVEN LAKAAKEMLA NSVKKKCSET FTNKLKEKHT DLGKEGVTDA
201 DAKEAILKTQ GTKTKGAEEL GKLFESVEVL SKAAKEMLAN SVKKKCSEEF
251 TAKLKGEHTD LGKEGVTDDN AKKAILKTNN DKTKGADELE KLFESVKQLS
301 KAAKEMLTNS VKKDCSEKFT TKLKDSHAEL GIQSVQDDNA KKAILKTHGT
351 KDKGAKELEE LFKSLESLSK AAQAALTNSV KKKCSEEFTN KLKSGHADLG
401 KQDATDDHAK AAILKTHATT DKGAKEFKDL FESVEGLLKA AQVALTNSVK
451 ELTSPVVAES PKKN;
- antigène recombinant polyépitopique chimérique ayant une séquence ayant au moins 95% d'identité avec la séquence (SEQ ID NO. 15) 
1 MGSSHHHHHH SSGLVPRGSH MASMTGGQQM GRGSLKVTEG TVVLSKHIPN
51 SGEITVELND SNSTQATKKT GKWDSNTSTL TISVNSKKTK NIVFTKEDTI
101 TVQKYDSAGT NLEGNLEVKE GTVTLSKEIA KSGEVTVALN DTNTTQATKK
151 TGAWDSKTST LTISVNSKKT TQLVFTKQDT ITVQKYDSAG TNLEGTLKVT
201 EGTVTLSKNI SKSGEITVAL DDTDSSGNKK SGTWDSGTST LTISKNRTKT
251 KQLVFTKEDT ITVQNYDSAG TNLEGKTLKV TEGTVVLSKN ILKSGEITVA
301 LDDSDTTQAT KKTGKWDSKT STLTISVNSQ KTKNLVFTKE DTITVQKYDS
351 AGTNLEGKLV VKEGTVTLSK NISKSGEVSV ELNDTDSSAA TKKTAAWNSG
401 TSTLTITVNS KKTKDLVFTK ENTITVQQYD SNGTKLEGSL TVTEGTVTLS
451 KNISKSGEIT VALNDTETTP ADKKTGEWKS DTSTLTISKN SQKPKQLVFT
501 KENTITVQNY NRAGNALEGS LTVEAGTVTL SKNISESGEI TVELKDTETT
551 PADKKSGTWD SKTSTLTISK NSQKTKQLVF TKENTITVQK YNTAGTKLEG
601 SAVEITKLDE IKNALKKDLE ALKAALK
- et au moins un adjuvant et/ou ferritine II.

2. Vaccin selon la revendication 1, **caractérisé en ce que** les antigènes polyépitopiques et/ou l'adjuvant et/ou le ferritine II sont liés sur des supports micro- ou nanoparticulaires contenant un composant métallochélateur pour la liaison desdits composants.

3. Vaccin selon l'une quelconque des revendications précédentes, **caractérisé en ce que** l'adjuvant est choisi dans le groupe comprenant le phosphate d'aluminium, l'hydroxyde d'aluminium, l'alum de potassium, un adjuvant moléculaire à base de normuramyl glycopeptides ou de leurs formes glycosylées, et un adjuvant sur la base de dispersion de particules préparées à partir de polymères biocompatibles.

4. Vaccin selon la revendication 3, où l'adjuvant est choisi dans le groupe comprenant le phosphate d'aluminium, l'hydroxyde d'aluminium, l'alum de potassium, de préférence l'adjuvant est l'hydroxyde d'aluminium.

5. Vaccin selon l'une quelconque des revendications précédentes pour la utilisation dans un procédé de prévention de la borréliose de Lyme en médecine vétérinaire et/ou humaine.
